# EUROPEAN PATENT APPLICATION

(11) **EP 4 385 977 A1**
(43) Date of publication of application: **19.06.2024**
(21) Application number: 22855860.7
(22) Date of filing: 08.08.2022
(51) Int. Cl.: C07D 215/12

(54) **DIFLUOROMETHYLATION REACTION OF HETEROCYCLIC RING USING TETRAFLUOROETHYLENE**

(30) Priority: 11.08.2021 JP 2021131010
(71) Applicant: AGC INC., Chiyoda-ku, Tokyo 1008405 (JP)
(72) Inventor: NOMURA, Yoshitaka, Tokyo 100-8405 (JP); SUZUKI, Haruya, Tokyo 100-8405 (JP)
(74) Representative: Müller-Boré & Partner Patentanwälte PartG mbB
(86) International application number: PCT/JP2022/030219
(87) International publication number: WO 2023/017798

(57) **Abstract**

The aim of the present invention is to provide an easy and inexpensive method for producing difluoromethyl-substituted aromatic heterocyclic compounds in good yield. Furthermore, the aim of the present invention is to provide an easy and inexpensive method for producing aromatic heterocycle-substituted difluoroacetic acid derivatives in good yield.

The present invention relates to a method for producing a difluoromethyl-substituted aromatic heterocyclic compound having a partial structure represented by the formula (IIa), which comprises reacting an N-oxido aromatic heterocyclic compound having a partial structure represented by the formula (Ia) with tetrafluoroethylene in the presence of a base, in a solvent selected from an aromatic hydrocarbon solvent, an ester solvent and an ether solvent. 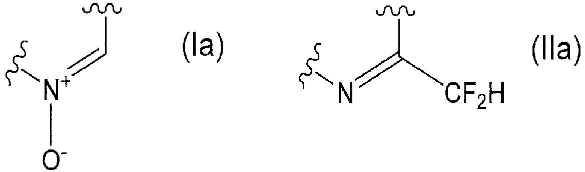 wherein each symbol is as described in the description.

Furthermore, the present invention relates to a method for producing an aromatic heterocycle-substituted difluoroacetic acid derivative having a partial structure represented by the formula (IIIb), which comprises reacting an N-oxido aromatic heterocyclic compound having a partial structure represented by the formula (Ib) with tetrafluoroethylene in the presence of a compound represented by the formula (IIb): R-YH and a base, in a solvent selected from an aromatic hydrocarbon solvent, an ester solvent and an ether solvent. 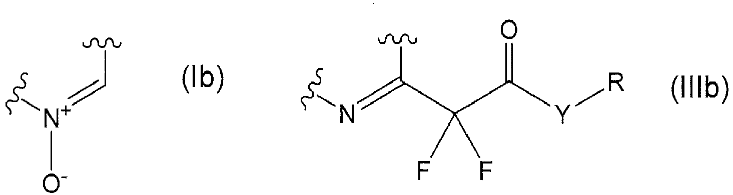 wherein each symbol is as described in the description.

## Description

### Technical Field

The present invention relates to a method for producing difluoromethyl-substituted aromatic heterocyclic compounds. Furthermore, the present invention relates to a method for producing aromatic heterocycle-substituted difluoroacetic acid derivatives.

### Background Art

Fluorine-containing alkyl group-substituted aromatic heterocyclic compounds, especially difluoromethyl-substituted aromatic heterocyclic compounds are useful as synthetic intermediates for pharmaceutical or agrochemical products.

The following methods are known as methods for producing fluorine-containing alkyl group-substituted aromatic heterocyclic compounds:
a method of reacting quinoline N-oxide with hexafluoropropene (CF₂=CFCF₃) in N,N-dimethylformamide and water at room temperature in one or two steps to produce 2-(1,2,2,2-tetrafluoroethyl)quinoline (Non-Patent Document 1, Scheme 5); and
a method of reacting quinoline N-oxide with hexafluoropropene (CF₂=CFCF₃) in N,N-dimethylformamide at room temperature to produce 2-(1,2,2,2-tetrafluoroethyl)quinoline (Non-Patent Document 2, Scheme 1 and Table 1).

However, none of the documents describe productions of difluoromethyl-substituted aromatic heterocyclic compounds or reactions with tetrafluoroethylene (CF₂=CF₂).

Aromatic heterocycle-substituted difluoroacetic acid derivatives are useful as synthetic intermediates for pharmaceutical or agrochemical products.

As methods for producing aromatic heterocycle-substituted fluoroalkyl carboxylic acid derivatives, for example, Non-Patent Document 1 discloses the following methods:
a method of reacting various aromatic heterocycle N-oxides with hexafluoropropene (CF₂=CFCF₃) and methanol in N,N-dimethylformamide to produce methyl 2-(2-heteroaryl)perfluoropropionate in two steps (Scheme 5, Table 2),
a method of reacting various aromatic heterocycle N-oxides with hexafluoropropene (CF₂=CFCF₃) and an amine in N,N-dimethylformamide to produce 2-(2-heteroaryl)perfluoropropionamide in two steps (Scheme 12, 13), and
a method of reacting quinoline N-oxide with chlorotrifluoroethylene (CF₂=CFCl) and methanol in N,N-dimethylformamide at 100°C to produce methyl 2-chloro-2-fluoro-2-(2-quinolyl)acetate (Scheme 17).

However, none of the documents describe productions of aromatic heterocycle-substituted difluoroacetic acid derivatives or reactions with tetrafluoroethylene (CF₂=CF₂).

### Document List

### Non-Patent Document

[Non-Patent Document 1] Chem. Eur. J. 2008, vol.14, pp 2577-2589
[Non-Patent Document 2] Mendeleev Commun. 2006, pp 161-163

### Summary of the Invention

### Problems to be Solved by the Invention

The aim of the present invention is to provide an easy and inexpensive method for producing difluoromethyl-substituted aromatic heterocyclic compounds in good yield.

Furthermore, the aim of the present invention is to provide an easy and inexpensive method for producing aromatic heterocycle-substituted difluoroacetic acid derivatives in good yield.

### Means of Solving the Problems

The present inventors have conducted intensive studies in an attempt to solve the above-mentioned problems and found that by reacting an N-oxido aromatic heterocyclic compound having a partial structure represented by the following formula (Ia) with tetrafluoroethylene in the presence of a base, in a specific solvent, a difluoromethyl-substituted aromatic heterocyclic compound having a partial structure represented by the following formula (IIa) with few by-products can be easily and inexpensively produced in good yield in one step, which resulted in the completion of the present invention.

Accordingly, the present invention provides the following (Invention a).
[1a] A method for producing a difluoromethyl-substituted aromatic heterocyclic compound having a partial structure represented by the formula (IIa): 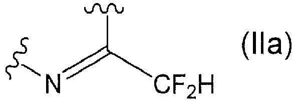 (hereinafter, to be referred to as difluoromethyl-substituted aromatic heterocyclic compound (IIa)), which comprises reacting an N-oxido aromatic heterocyclic compound having a partial structure represented by the formula (Ia): 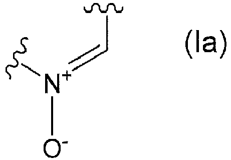 (hereinafter, to be referred to as N-oxido aromatic heterocyclic compound (Ia)) with tetrafluoroethylene in the presence of a base, in a solvent selected from an aromatic hydrocarbon solvent, an ester solvent and an ether solvent.
[2a] The method according to the above [1a], wherein the reaction is carried out at temperature in the range of 100 to 300°C.
[3a] The method according to the above [1a] or [2a], wherein the reaction is carried out under pressure in the range of 0.1 to 10.0 MPa.
[4a] The method according to any of the above [1a] to [3a], wherein the reaction is carried out in the presence of a buffer solution with pH 5.0 to 8.0.
[5a] The method according to any of the above [1a] to [4a], wherein the N-oxido aromatic heterocyclic compound having the partial structure represented by the formula (Ia) is an N-oxido aromatic heterocyclic compound represented by the formula (IA) or (IB): 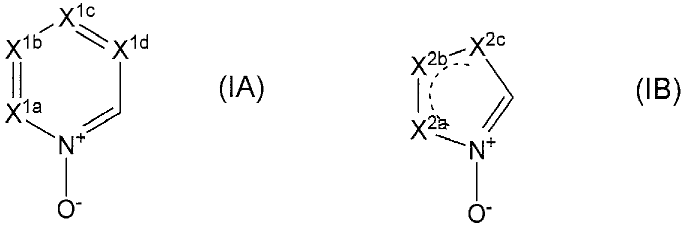 wherein
   X^{1a} is CR^{1a} or N;
   X^{1b} is CR^{1b} or N;
   X^{1c} is CR^{1c} or N;
   X^{1d} is CR^{1d} or N;
   R^{1a}, R^{1b}, R^{1c} and R^{1d} are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group, or R^{1a} and R^{1b}, R^{1b} and R^{1c}, or R^{1c} and R^{1d} are optionally taken together to form a C₆₋₁₀ arene optionally substituted by substituent(s) selected from a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group;
   X^{2a} is CR^{2a}, N, NR^{2a}, S or O;
   X^{2b} is CR^{2b} or N;
   X^{2c} is CR^{2c}, N, NR^{2c}, S or O; and
   R^{2a}, R^{2b} and R^{2c} are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group, or R^{2a} and R^{2b}, or R^{2b} and R^{2c} are optionally taken together to form a C₆₋₁₀ arene optionally substituted by substituent(s) selected from a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group
      (hereinafter, to be referred to as N-oxido aromatic heterocyclic compound (IA) or (IB)).
[6a] The method according to the above [5a], wherein the N-oxido aromatic heterocyclic compound having the partial structure represented by the formula (Ia) is represented by the formula (IA), and R^{1a} and R^{1b}, R^{1b} and R^{1c}, or R^{1c} and R^{1d} in the formula are taken together to form a C₆₋₁₀ arene optionally substituted by substituent(s) selected from a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group.
[7a] The method according to the above [5a], wherein the N-oxido aromatic heterocyclic compound having the partial structure represented by the formula (Ia) is represented by the formula (IA), and R^{1a}, R^{1b}, R^{1c} and R^{1d} in the formula are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group.
[8a] The method according to the above [5a], wherein the N-oxido aromatic heterocyclic compound having the partial structure represented by the formula (Ia) is represented by the formula (IB), and R^{2a} and R^{2b}, or R^{2b} and R^{2c} in the formula are taken together to form a C₆₋₁₀ arene optionally substituted by substituent(s) selected from a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group.
[9a] The method according to the above [5a], wherein the N-oxido aromatic heterocyclic compound having the partial structure represented by the formula (Ia) is represented by the formula (IB), and R^{2a}, R^{2b} and R^{2c} in the formula are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group.

Furthermore, the present inventors have conducted intensive studies in an attempt to solve the above-mentioned problems and found that by reacting an N-oxido aromatic heterocyclic compound having a partial structure represented by the following formula (Ib) with tetrafluoroethylene in the presence of a compound represented by the following formula (IIb) and a base, in a specific solvent, an aromatic heterocycle-substituted difluoroacetic acid derivative having a partial structure represented by the following formula (IIIb) with few by-products can be easily and inexpensively produced in good yield in one step, which resulted in the completion of the present invention.

Accordingly, the present invention provides the following (Invention b).
[1b] A method for producing an aromatic heterocycle-substituted difluoroacetic acid derivative having a partial structure represented by the formula (IIIb): 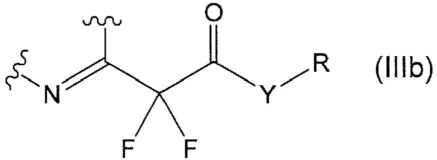 wherein
   R is a C₁₋₆ alkyl group, a C₃₋₈ cycloalkyl group, a C₇₋₁₆ aralkyl group or a C₆₋₁₀ aryl group, wherein the C₁₋₈ alkyl group is optionally substituted by substituent(s) selected from a halogen atom, a cyano group, a C₁₋₆ alkoxy group and a C₁₋₆ haloalkoxy group, and the C₃₋₈ cycloalkyl group, C₇₋₁₆ aralkyl group and C₆₋₁₀ aryl group are each optionally substituted by substituent(s) selected from a halogen atom, a cyano group, a C₁₋₆ alkyl group, a C₁₋₆ haloalkyl group, a C₁₋₆ alkoxy group and a C₁₋₆ haloalkoxy group,
   Y is O, S or NR^{Y}, and
   R^{Y} is a hydrogen atom or a C₁₋₈ alkyl group, or R^{Y} and R are optionally taken together to form a nitrogen-containing heterocycle with the nitrogen atom to which they are bond (hereinafter, to be referred to as aromatic heterocycle-substituted difluoroacetic acid derivative (IIIb)),
   which comprises reacting an N-oxido aromatic heterocyclic compound having a partial structure represented by the formula (Ib) : 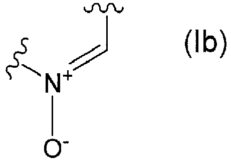 (hereinafter, to be referred to as N-oxido aromatic heterocyclic compound (Ib)) with tetrafluoroethylene in the presence of a compound represented by the formula (IIb): R-YH wherein each symbol in the formula is as defined above (hereinafter, to be referred to as compound (IIb)) and a base, in a solvent selected from an aromatic hydrocarbon solvent, an ester solvent and an ether solvent.
[2b] The method according to the above [1b], wherein the reaction is carried out at temperature in the range of 100 to 300°C.
[3b] The method according to the above [1b] or [2b], wherein the reaction is carried out under pressure in the range of 0.1 to 10.0 MPa.
[4b] The method according to any of the above [1b] to [3b], wherein the N-oxido aromatic heterocyclic compound having the partial structure represented by the formula (Ib) is an N-oxido aromatic heterocyclic compound represented by the formula (IA) or (IB): 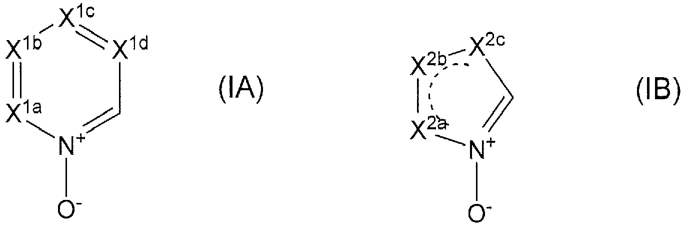 wherein
   X^{1a} is CR^{1a} or N;
   X^{1b} is CR^{1b} or N;
   X^{1c} is CR^{1c} or N;
   X^{1d} is CR^{1d} or N;
   R^{1a}, R^{1b}, R^{1c} and R^{1d} are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group, or R^{1a} and R^{1b}, R^{1b} and R^{1c}, or R^{1c} and R^{1d} are optionally taken together to form a C₆₋₁₀ arene optionally substituted by substituent(s) selected from a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group;
   X^{2a} is CR^{2a}, N, NR^{2a}, S or O;
   X^{2b} is CR^{2b} or N;
   X^{2c} is CR^{2c}, N, NR^{2c}, S or O; and
   R^{2a}, R^{2b} and R^{2c} are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group, or R^{2a} and R^{2b}, or R^{2b} and R^{2c} are optionally taken together to form a C₆₋₁₀ arene optionally substituted by substituent(s) selected from a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group (hereinafter, to be referred to as N-oxido aromatic heterocyclic compound (IA) or (IB)).
[5b] The method according to the above [4b], wherein the N-oxido aromatic heterocyclic compound having the partial structure represented by the formula (Ib) is represented by the formula (IA), and R^{1a} and R^{1b}, R^{1b} and R^{1c}, or R^{1c} and R^{1d} in the formula are taken together to form a C₆₋₁₀ arene optionally substituted by substituent(s) selected from a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group.
[6b] The method according to the above [4b], wherein the N-oxido aromatic heterocyclic compound having the partial structure represented by the formula (Ib) is represented by the formula (IA), and R^{1a}, R^{1b}, R^{1c} and R^{1d} in the formula are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group.
[7b] The method according to the above [4b], wherein the N-oxido aromatic heterocyclic compound having the partial structure represented by the formula (Ib) is represented by the formula (IB), and R^{2a} and R^{2b}, or R^{2b} and R^{2c} in the formula are taken together to form a C₆₋₁₀ arene optionally substituted by substituent(s) selected from a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group.
[8b] The method according to the above [4b], wherein the N-oxido aromatic heterocyclic compound having the partial structure represented by the formula (Ib) is represented by the formula (IB), and R^{2a}, R^{2b} and R^{2c} in the formula are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group.

### Effect of the Invention

According to the present invention (Invention a), by an easy and inexpensive method of flowing tetrafluoroethylene into N-oxido aromatic heterocyclic compound (Ia) in the presence of a base, in a specific solvent, difluoromethyl-substituted aromatic heterocyclic compound (IIa) with few by-products can be produced in good yield in one step.

Furthermore, according to the present invention (Invention b), by an easy and inexpensive method of flowing tetrafluoroethylene into N-oxido aromatic heterocyclic compound (Ib) in the presence of compound (IIb) and a base, in a specific solvent, aromatic heterocycle-substituted difluoroacetic acid derivative (IIIb) with few by-products can be produced in good yield in one step.

### Description of Embodiments

Hereinafter, the definitions of the groups used herein will be described in detail. Unless otherwise specified, the groups have the following definitions.

As used herein, the compound represented by the formula is indicated by adding the formula number to the "Compound". For example, the compound represented by the formula (1) is indicated as "Compound (1)".

As used herein, the numerical range represented by "to" or "-" means a numerical range where the numbers before and after "to" or "-" are the lower and upper limits.

As used herein, when the element symbol "C" with a numerical range by numbers before and after "-" is given for the name of an arbitrary group, it means each arbitrary group having an integer number of carbon atoms where the numbers before and after the "-" are the lower and upper limits. For example, an alkyl group having 1 to 3 carbon atoms may be referred to as a "C₁₋₃ alkyl group", which indicates each of - CH₃, -C₂H₅, -C₃H₇ and the like. The same applies to other groups.

As used herein, the "halogen atom" means a fluorine atom, a chlorine atom, a bromine atom or an iodine atom.

As used herein, the "C₁₋₈ alkyl group" means a linear or branched saturated hydrocarbon group having 1 to 8 carbon atoms. Examples of the "C₁₋₈ alkyl group" include methyl, ethyl, propyl, isopropyl, butyl, isobutyl, sec-butyl, tert-butyl, pentyl, isopentyl, neo-pentyl, 1-ethylpropyl, hexyl, isohexyl, 1,1-dimethylbutyl, 2,2-dimethylbutyl, 3,3-dimethylbutyl, 2-ethylbutyl, heptyl, octyl and the like. The preferred is a C₁₋₄ alkyl group.

As used herein, the "C₁₋₆ alkyl group" means a linear or branched saturated hydrocarbon group having 1 to 6 carbon atoms. Examples of the "C₁₋₆ alkyl group" include methyl, ethyl, propyl, isopropyl, butyl, isobutyl, sec-butyl, tert-butyl, pentyl, isopentyl, neo-pentyl, 1-ethylpropyl, hexyl, isohexyl, 1,1-dimethylbutyl, 2,2-dimethylbutyl, 3,3-dimethylbutyl, 2-ethylbutyl and the like. The preferred is a C₁₋₄ alkyl group.

As used herein, the "C₁₋₄ alkyl group" means a linear or branched saturated hydrocarbon group having 1 to 4 carbon atoms. Examples of the "C₁₋₄ alkyl group" include methyl, ethyl, propyl, isopropyl, butyl, isobutyl, sec-butyl and tert-butyl.

As used herein, the "C₁₋₂₀ alkyl group" means a linear or branched saturated hydrocarbon group having 1 to 20 carbon atoms. Examples of the "C₁₋₂₀ alkyl group" include methyl, ethyl, propyl, isopropyl, butyl, isobutyl, sec-butyl, tert-butyl, pentyl, hexyl, octyl, decyl, stearyl (octadecyl), icosyl and the like.

As used herein, the "C₂₋₂₀ alkenyl group" means a linear or branched unsaturated hydrocarbon group having 2 to 20 carbon atoms and at least one double bond. Examples of the "C₂₋₂₀ alkenyl group" include ethenyl, 1-propenyl, 2-propenyl, 1-butenyl, 2-butenyl, 3-butenyl, 1-pentenyl, 1-hexenyl, 1-octenyl, 1-decenyl, 1-octadecenyl, 1-icosenyl and the like.

As used herein, the "C₂₋₂₀ alkynyl group" means a linear or branched unsaturated hydrocarbon group having 2 to 20 carbon atoms and at least one triple bond. Examples of the "C₂₋₂₀ alkynyl group" include ethynyl, 1-propynyl, 2-propynyl, 1-butynyl, 2-butynyl, 3-butynyl, 1-pentynyl, 1-hexynyl, 1-octynyl, 1-decynyl, 1-octadecynyl, 1-icosynyl and the like.

As used herein, the "C₁₋₂₀ alkylidene group" means a divalent saturated hydrocarbon group resulting from the removal of two hydrogen atoms from the same carbon atom of a linear or branched saturated hydrocarbon having 1 to 20 carbon atoms. Examples of the "C₁₋₂₀ alkylidene group" include methylidene, ethylidene, propylidene, butylidene, pentylidene, hexylidene, octylidene, decylidene, octadecylidene, icosylidene and the like.

As used herein, the "C₂₋₂₀ alkenylidene group" means a divalent unsaturated hydrocarbon group resulting from the removal of two hydrogen atoms from the same carbon atom of a linear or branched unsaturated hydrocarbon having 2 to 20 carbon atoms and at least one double bond. Examples of the "C₂₋₂₀ alkenylidene group" include ethenylidene, propenylidene, butenylidene, pentenylidene, hexenylidene, octenylidene, decenylidene, octadecenylidene, icosenylidene and the like.

As used herein, the "C₃₋₂₀ alkynylidene group" means a divalent unsaturated hydrocarbon group resulting from the removal of two hydrogen atoms from the same carbon atom of a linear or branched unsaturated hydrocarbon having 3 to 20 carbon atoms and at least one triple bond. Examples of the "C₃₋₂₀ alkynylidene group" include propynylidene, butynylidene, pentynylidene, hexynylidene, octynylidene, decynylidene, octadecynylidene, icosynylidene and the like.

As used herein, the "C₁₋₆ alkoxy group" means a group represented by the formula R¹¹O- wherein R¹¹ is a C₁₋₆ alkyl group. Examples of the "C₁₋₆ alkoxy group" include methoxy, ethoxy, propoxy, isopropoxy, butyloxy, isobutyloxy, secbutyloxy, tert-butyloxy, pentyloxy, hexyloxy and the like. The preferred is a C₁₋₄ alkoxy group.

As used herein, the "C₁₋₆ haloalkyl group" means a group in which one or more hydrogen atoms of the "C₁₋₆ alkyl group" are replaced with halogen atoms. Examples of the "C₁₋₆ haloalkyl group" include fluoromethyl, 2-fluoroethyl, 3-fluoropropyl, 4-fluorobutyl, trifluoromethyl, difluoromethyl, perfluoroethyl, perfluoropropyl, chloromethyl, 2-chloroethyl, bromomethyl, 2-bromoethyl, iodomethyl, 2-iodoethyl and the like. The preferred is trifluoromethyl.

As used herein, the "C₁₋₆ haloalkoxy group" means a group in which one or more hydrogen atoms of the "C₁₋₆ alkoxy group" are replaced with halogen atoms. Examples of the "C₁₋₆ haloalkoxy group" include bromomethoxy, 2-bromoethoxy, 3-bromopropoxy, 4-bromobutoxy, iodomethoxy, 2-iodoethoxy, 3-iodopropoxy, 4-iodobutoxy, fluoromethoxy, 2-fluoroethoxy, 3-fluoropropoxy, 4-fluorobutoxy, tribromomethoxy, trichloromethoxy, trifluoromethoxy, difluoromethoxy, perfluoroethoxy, perfluoropropoxy, perfluoroisopropoxy, 1,1,2,2-tetrafluoroethoxy and 2-chloro-1,1,2-trifluoroethoxy.

As used herein, the "C₁₋₆ alkylsulfanyl group" means a group represented by the formula R¹¹S- wherein R¹¹ is a C₁₋₆ alkyl group. Examples of the "C₁₋₆ alkylsulfanyl group" include methylsulfanyl, ethylsulfanyl, propylsulfanyl, isopropylsulfanyl, butylsulfanyl, isobutylsulfanyl, secbutylsulfanyl, tert-butylsulfanyl, pentylsulfanyl, hexylsulfanyl and the like. The preferred is a C₁₋₄ sulfanyl group.

As used herein, the "mono-C₁₋₆ alkylamino group" means a group represented by the formula R¹¹NH- wherein R¹¹ is a C₁₋₆ alkyl group. Examples of the "mono-C₁₋₆ alkylamino group" include methylamino, ethylamino, propylamino, isopropylamino, butylamino, isobutylamino, sec-butylamino, tert-butylamino, pentylamino, hexylamino and the like. The preferred is a mono-C₁₋₄ alkylamino group.

As used herein, the "di-C₁₋₆ alkylamino group" means a group represented by the formula R¹¹₂N- wherein the two R¹¹ are each independently a C₁₋₆ alkyl group. Examples of the "di-C₁₋₆ alkylamino group" include dimethylamino, diethylamino, N-ethyl-N-methylamino, dipropylamino, diisopropylamino, dibutylamino, diisobutylamino, disec-butylamino, ditert-butylamino, dipentylamino, dihexylamino and the like. The preferred is a di-C₁₋₄ alkylamino group.

As used herein, the "C₁₋₆ alkoxy-carbonyl group" means a group represented by the formula R¹¹OC(=O)- wherein R¹¹ is a C₁₋₆ alkyl group. Examples of the "C₁₋₆ alkoxy-carbonyl group" include methoxycarbonyl, ethoxycarbonyl, propoxycarbonyl, isopropoxycarbonyl, butoxycarbonyl, isobutoxycarbonyl, secbutoxycarbonyl, tert-butoxycarbonyl, pentyloxycarbonyl, hexyloxycarbonyl and the like. The preferred is a C₁₋₄ alkoxy-carbonyl group.

As used herein, the "C₃₋₈ cycloalkyl group" means a cyclic saturated hydrocarbon group having 3 to 8 carbon atoms. Examples of the "C₃₋₈ cycloalkyl group include cyclopropyl, cyclobutyl, cyclopentyl, cyclohexyl, cycloheptyl, and cyclooctyl.

As used herein, the "C₃₋₈ cycloalkenyl group" means a cyclic unsaturated hydrocarbon group having 3 to 8 carbon atoms. Examples of the "C₃₋₈ cycloalkenyl group include cyclopropenyl, cyclobutenyl, cyclopentenyl, cyclohexenyl, cycloheptenyl, and cyclooctenyl.

As used herein, the "C₃₋₈ cycloalkylidene group" means a divalent saturated hydrocarbon resulting from the removal of two hydrogen atoms from the same carbon atom of a cyclic saturated hydrocarbon having 3 to 8 carbon atoms. Examples of the "C₃₋₈ cycloalkylidene group include cyclopropylidene, cyclobutylidene, cyclopentylidene, cyclohexylidene, cycloheptylidene, and cyclooctylidene.

As used herein, the "C₃₋₈ cycloalkenylidene group" means a divalent unsaturated hydrocarbon resulting from the removal of two hydrogen atoms from the same carbon atom of a cyclic unsaturated hydrocarbon having 3 to 8 carbon atoms. Examples of the "C₃₋₈ cycloalkenylidene group include cyclopropenylidene, cyclobutenylidene, cyclopentenylidene, cyclohexenylidene, cycloheptenylidene, and cyclooctenylidene.

As used herein, the "C₆₋₁₀ aryl group" means a hydrocarbon group having 6 to 10 carbon atoms and having aromaticity. Examples of the "C₆₋₁₀ aryl group" include phenyl, 1-naphthyl and 2-naphthyl. The preferred is phenyl.

As used herein, the "C₇₋₁₆ aralkyl group" means the "C₁₋₆ alkyl group" substituted by the "C₆₋₁₀ aryl group". Examples of the "C₇₋₁₆ aralkyl group" include benzyl, 1-phenylethyl, 2-phenylethyl, 3-phenylpropyl, 4-phenylbutyl, (1-naphthyl)methyl, (2-naphthyl)methyl and the like. The preferred is benzyl.

As used herein, the "C₆₋₁₀ arene" means a hydrocarbon ring having 6 to 10 carbon atoms and having aromaticity. Examples of the "C₆₋₁₀ arene" include benzene and naphthalene. The preferred is benzene.

As used herein, Examples of the substituent of the "optionally substituted C₆₋₁₀ arene" include a halogen atom, a cyano group, a C₁₋₆ alkyl group, a hydroxy group, a C₁₋₆ alkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group.

As used herein, the "heterocyclic group" means an aromatic or non-aromatic ring containing at least one hetero atom selected from a nitrogen atom, an oxygen atom and a sulfur atom.

Examples of the "aromatic heterocyclic group" include 5- or 6-membered monocyclic aromatic heterocyclic groups such as thienyl, furyl, pyrrolyl, imidazolyl, pyrazolyl, thiazolyl, isothiazolyl, oxazolyl, isoxazolyl, pyridyl, pyridone-yl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, triazinyl and the like; and 8- to 14-membered fused polycyclic (preferably bi- or tricyclic) aromatic heterocyclic groups such as benzothiophenyl, benzofuranyl, benzimidazolyl, benzoxazolyl, benzisoxazolyl, benzothiazolyl, benzisothiazolyl, benzotriazolyl, imidazopyridinyl, thienopyridinyl, furopyridinyl, pyrrolopyridinyl, pyrazolopyridinyl, oxazolopyridinyl, thiazolopyridinyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acridinyl, phenazinyl, phenothiazinyl, phenoxazinyl and the like.

Examples of the "non-aromatic heterocyclic group" include 3- to 8-membered monocyclic non-aromatic heterocyclic groups such as aziridinyl, oxiranyl, thiiranyl, azetidinyl, oxetanyl, thietanyl, tetrahydrothienyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisoxazolyl, piperidinyl, piperazinyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, diazocanyl and the like; and 9- to 14-membered fused polycyclic (preferably bi- or tricyclic) non-aromatic heterocyclic groups such as dihydrobenzofuranyl, dihydrobenzimidazolyl, dihydrobenzoxazolyl, dihydrobenzothiazolyl, dihydrobenzisothiazolyl, dihydronaphtho[2,3-b]thienyl, tetrahydroisoquinolyl, tetrahydroquinolyl, 4H-quinolizinyl, indolinyl, isoindolinyl, tetrahydrothieno[2,3-c]pyridinyl, tetrahydrobenzazepinyl, tetrahydroquinoxalinyl, tetrahydrophenanthridinyl, hexahydrophenothiazinyl, hexahydrophenoxazinyl, tetrahydrophthalazinyl, tetrahydronaphthyridinyl, tetrahydroquinazolinyl, tetrahydrocinnolinyl, tetrahydrocarbazolyl, tetrahydro-β-carbolinyl, tetrahydroacridinyl, tetrahydrophenazinyl, tetrahydrothioxanthenyl, octahydroisoquinolyl and the like.

As used herein, the "nitrogen-containing heterocycle" means a ring containing at least one nitrogen atom, and optionally containing additional hetero atom(s) selected from a nitrogen atom, an oxygen atom and a sulfur atom. Examples of the "nitrogen-containing heterocycle" include aziridine, azetidine, pyrrolidine, piperidine, piperazine, morpholine, thiomorpholine and the like. The preferred is a 3-membered to 8-membered nitrogen-containing heterocycle, and the more preferred is a 5-membered or 6-membered nitrogen-containing heterocycle.

As used herein, the "monovalent organic group" means a monovalent group containing carbon atom(s) as essential. Examples of the "monovalent organic group" include hydrocarbon groups such as a C₁₋₂₀ alkyl group, a C₂₋₂₀ alkenyl group, a C₂₋₂₀ alkynyl group, a C₃₋₈ cycloalkyl group, a C₃₋₈ cycloalkenyl group, a C₆₋₁₀ aryl group, a C₇₋₁₆ aralkyl group and the like; heterocyclic groups such as an aromatic heterocyclic group, a non-aromatic heterocyclic group and the like; and the like. The "monovalent organic group" also include those having substituent(s) such as a C₁₋₆ alkyl group, an amino group, a mono-C₁₋₆ alkylamino group, a di-C₁₋₆ alkylamino group and the like.

As used herein, the "divalent organic group" means a divalent group containing carbon atom(s) as essential and having two bonds from the same carbon atom. Examples of the "divalent organic group" include a C₁₋₂₀ alkylidene group, a C₂₋₂₀ alkenylidene group, a C₃₋₂₀ alkynylidene group, a C₃₋₈ cycloalkylidene group, and a C₃₋₈ cycloalkenylidene group. The "divalent organic group" also include those having substituent(s) such as a C₁₋₆ alkyl group, an amino group, a mono-C₁₋₆ alkylamino group, a di-C₁₋₆ alkylamino group and the like.

As used herein, examples of the "nitrogen-containing bicyclo ring" include 1,8-diazabicyclo[5.4.0]-7-undecene, 1,4-diazabicyclo[2.2.2]octane and the like.

As used herein, the "unsaturated nitrogen-containing heterocycle" means a ring containing at least one double bond and at least one nitrogen atom, and optionally containing additional hetero atom(s) selected from a nitrogen atom, an oxygen atom and a sulfur atom. Examples of the "unsaturated nitrogen-containing heterocycle" include pyridine, imidazole, quinoline, octahydropyrimido[1,2-a]azepine and the like.

As used herein, examples of the "counter anion" include a halide ion, a hydroxide ion and the like.

As used herein, examples of the "alkali metal" include lithium, potassium, sodium, cesium and the like.

Hereinafter, the production method of the present invention (Invention a) is explained.

In the present invention, difluoromethyl-substituted aromatic heterocyclic compound (IIa) is produced by reacting N-oxido aromatic heterocyclic compound (Ia) with tetrafluoroethylene in the presence of a base, in a solvent selected from an aromatic hydrocarbon solvent, an ester solvent and an ether solvent. For example, if a proton source is present and it is water, the reaction mechanism is as follows.

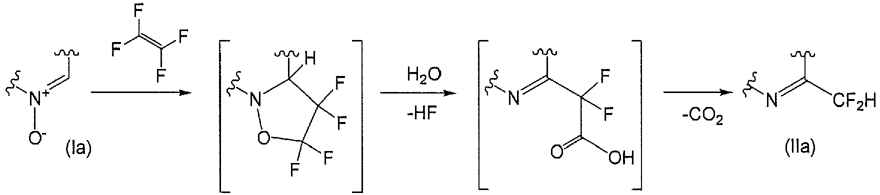

Specific examples of N-oxido aromatic heterocyclic compound (Ia) include the following N-oxido aromatic heterocyclic compounds (IA) and (IB): 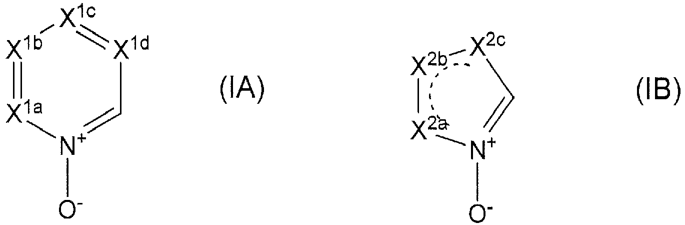 wherein
X^{1a} is CR^{1a} or N;
X^{1b} is CR^{1b} or N;
X^{1c} is CR^{1c} or N;
X^{1d} is CR^{1d} or N;
R^{1a}, R^{1b}, R^{1c} and R^{1d} are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group, or R^{1a} and R^{1b}, R^{1b} and R^{1c}, or R^{1c} and R^{1d} are optionally taken together to form a C₆₋₁₀ arene optionally substituted by substituent(s) selected from a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group;
X^{2a} is CR^{2a}, N, NR^{2a}, S or O;
X^{2b} is CR^{2b} or N;
X^{2c} is CR^{2c}, N, NR^{2c}, S or O; and
R^{2a}, R^{2b} and R^{2c} are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group, or R^{2a} and R^{2b}, or R^{2b} and R^{2c} are optionally taken together to form a C₆₋₁₀ arene optionally substituted by substituent(s) selected from a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group.

R^{1a}, R^{1b}, R^{1c} and R^{1d} are preferably each independently a hydrogen atom, a cyano group or a C₁₋₆ alkyl group, or R^{1a} and R^{1b}, R^{1b} and R^{1c}, or R^{1c} and R^{1d} are optionally taken together to form a C₆₋₁₀ arene optionally substituted by C₁₋₆ alkoxy-carbonyl group (s) .

R^{2a}, R^{2b} and R^{2c} are preferably each independently a hydrogen atom, a C₁₋₆ alkyl group or a C₇₋₁₆ aralkyl group, or R^{2a} and R^{2b}, or R^{2b} and R^{2c} are optionally taken together to form a C₆₋₁₀ arene.

In the formula (IB), one of the bond R^{2a}-R^{2b} and the bond R^{2b}-R^{2c} is a single bond, and the other is a double bond. Provided that when X^{2a} is S or O, then the bond R^{2a}-R^{2b} is a single bond, and the bond R^{2b}-R^{2c} is a double bond. Also, provided that when X^{2c} is S or O, then the bond R^{2a}-R^{2b} is a double bond, and the bond R^{2b}-R^{2c} is a single bond.

Specific examples of N-oxido aromatic heterocyclic compound (IA) include the following N-oxido aromatic heterocyclic compounds (IA-a) - (IA-e). 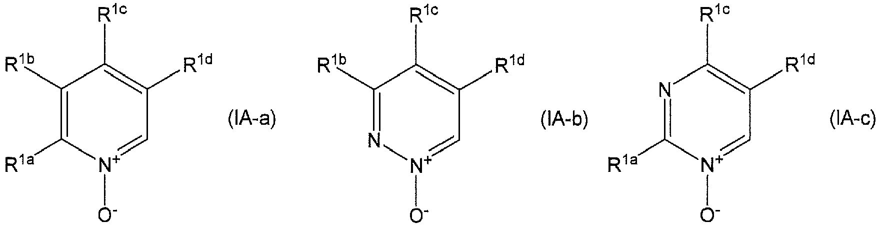 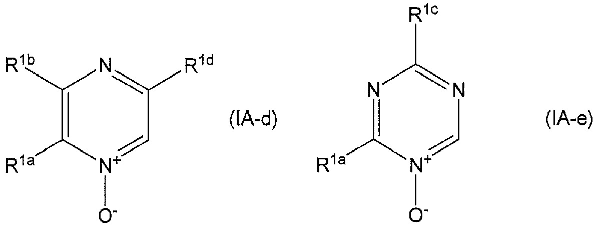 wherein each symbol in the formula is as defined above.

Among them, the preferred are (IA-a), (IA-b) and (IA-d).

Preferable specific examples of N-oxido aromatic heterocyclic compound (IA) include the following compounds. 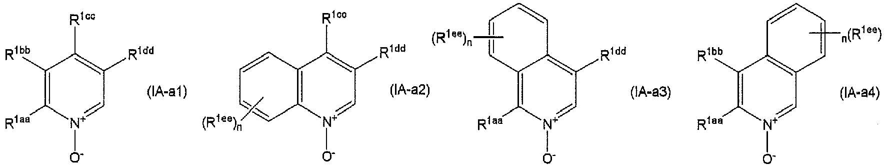 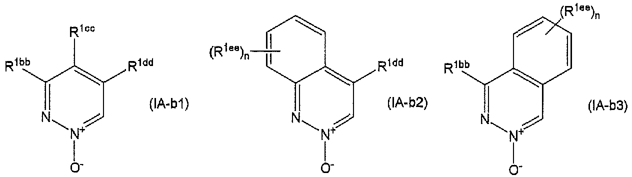 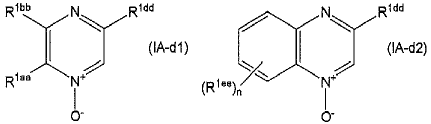 wherein
R^{1aa}, R^{1bb}, R^{1cc} and R^{1dd} are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group;
R^{1ee} in the number of n are each independently a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group; and
n is an integer of 0 to 4.
   R^{1aa}, R^{1bb}, R^{1cc} and R^{1dd} are preferably each independently a hydrogen atom, a cyano group or a C₁₋₆ alkyl group;
R^{1ee} in the number of n are preferably each independently a C₁₋₆ alkoxy-carbonyl group; and
n is preferably 0 or 1.

Specific examples of N-oxido aromatic heterocyclic compound (IB) include the following N-oxido aromatic heterocyclic compounds (IB-a) - (IB-ff). 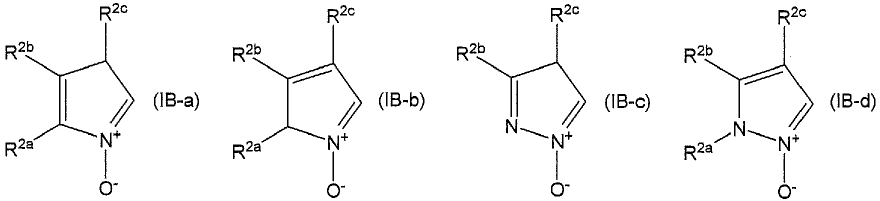 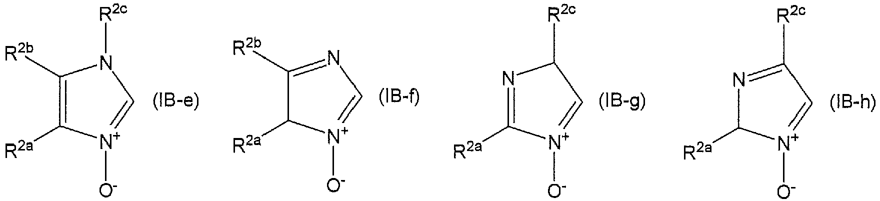 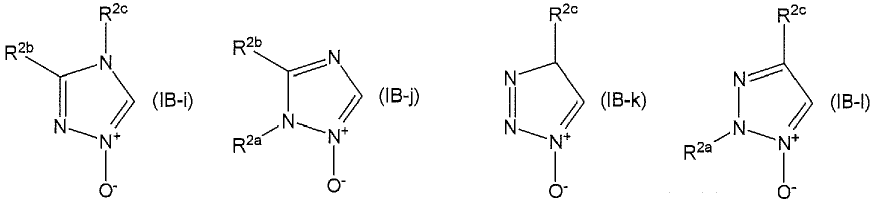 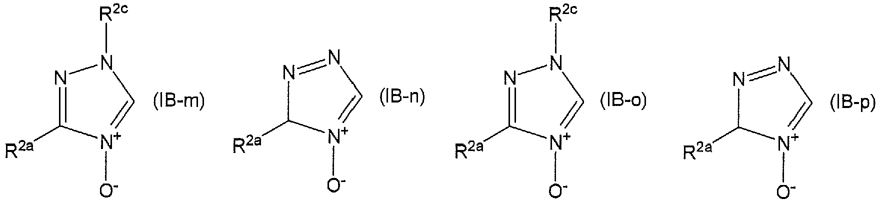 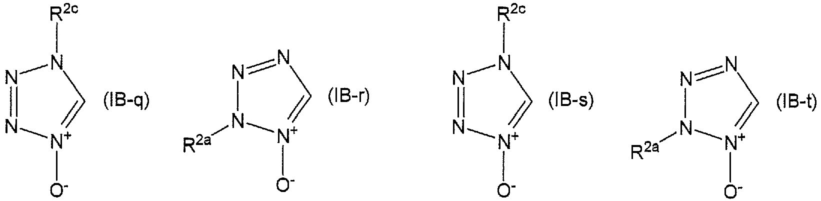 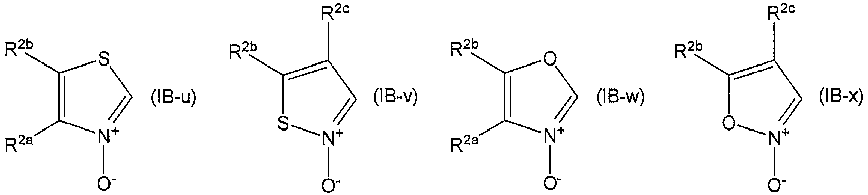 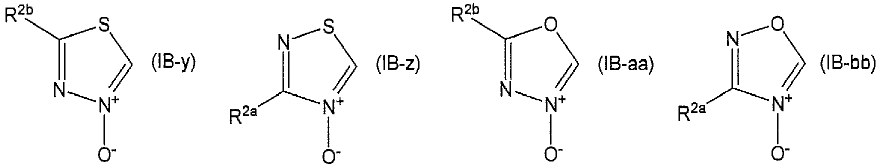 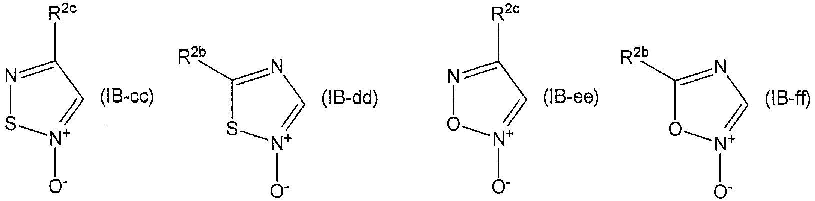 wherein each symbol in the formula is as defined above.

Among them, the preferred are (IB-e), (IB-u) and (IB-w).

Preferable specific examples of N-oxido aromatic heterocyclic compound (IB) include the following compounds. 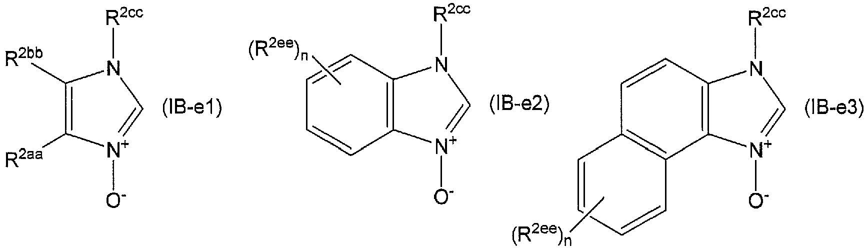 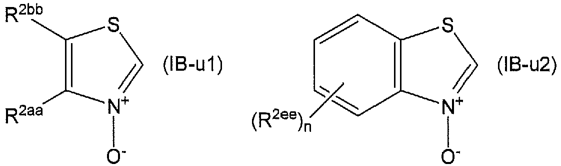 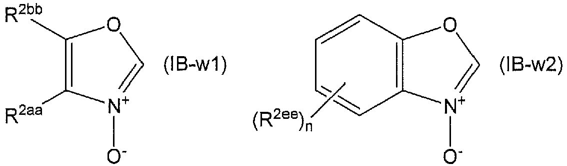 wherein
R^{2aa}, R^{2bb} and R^{2cc} are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group;
R^{2ee} in the number of n are each independently a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group; and
n is an integer of 0 to 4.

R^{2aa}, R^{2bb} and R^{2cc} are preferably each independently a hydrogen atom, a C₁₋₆ alkyl group or a C₇₋₁₆ aralkyl group; and n is preferably 0.

N-Oxido aromatic heterocyclic compound (Ia) may be commercially available, or can be produced according to a method known per se.

Tetrafluoroethylene (CF₂=CF₂) can be produced according to a known method. Since tetrafluoroethylene is in a gaseous state at room temperature under atmospheric pressure, it is added to the reaction system by inflow. In this case, tetrafluoroethylene diluted with nitrogen gas may be flowed into the reaction system. The amount of the tetrafluoroethylene to be used is generally 1 to 100 mol, preferably 1 to 10 mol, per 1 mol of N-oxido aromatic heterocyclic compound (Ia).

The reaction is carried out using a proton source, and the proton source may be present from the start of the reaction, or added during the reaction, or it may be a compound generated in the reaction process (e.g., hydrogen fluoride). From the aspect of the reaction rate, the proton source is preferably present from the start of the reaction.

Examples of the proton source include inorganic acids, organic acids, water and the like, specifically, hydrogen fluoride, hydrogen chloride, ammonium salts, water, buffer solutions and the like. Among them, the preferred are water and hydrogen fluoride. Water can be molecules hydrated in the raw material N-oxido aromatic heterocyclic compound (Ia). Hydrogen fluoride can be molecules generated in the reaction process.

The amount of the proton source to be used can be appropriately selected. When the proton source is, for example, water or a buffer solution as described above, the amount is preferably 0.05 to 0.5 times the volume of the solvent described below. When the proton source is, for example, hydrated water molecules as described above, the amount is preferably 1 to 10 mol, per 1 mol of N-oxido aromatic heterocyclic compound (Ia). When the proton source is, for example, hydrogen fluoride molecules generated in the reaction process, as described above, the amount is preferably 1 mol, per 1 mol of N-oxido aromatic heterocyclic compound (Ia).

The reaction is carried out in the presence of a base.

Through studies conducted by the present inventors, it has been found that a side reaction occurs during the following reaction in the above-mentioned reaction mechanism.

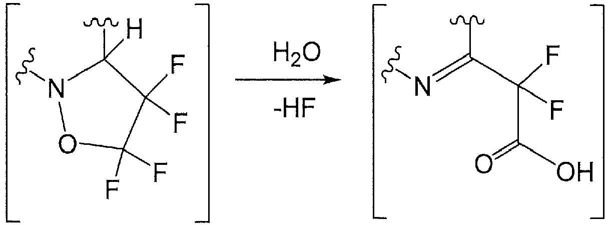

In order to solve this problem, in the present invention, a base is present in the reaction system to promote the withdrawal of protons from the above intermediate. This improves the reaction rate, reduces side reactions, and improves the yield.

As used herein, the base is a base capable of withdrawing a proton from the above intermediate, and examples of such base include a Brønsted base. It may be one that can be converted into a Brønsted base in the reaction system.

The above bases are broadly classified into N atom-containing bases and N atom-free bases.

Examples of the N atom-containing base include amine compounds, ammonium compounds, imine compounds, and iminium compounds.

The amine compound is, for example, a compound represented by the following formula (b1). 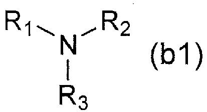 wherein
R₁, R₂ and R₃ are each independently a monovalent organic group, or R₁ and R₂ are optionally taken together to form an optionally substituted nitrogen-containing heterocycle, or R₁, R₂ and R₃ are optionally taken together to form a nitrogen-containing bicyclo ring.

The ammonium compound is, for example, a compound represented by the following formula (b2). 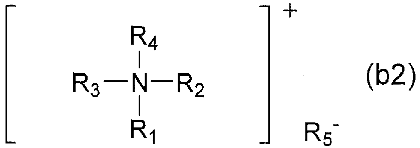 wherein
R₁, R₂ and R₃ are each independently a monovalent organic group, or R₁ and R₂ are optionally taken together to form an optionally substituted nitrogen-containing heterocycle, or R₁, R₂ and R₃ are optionally taken together to form a nitrogen-containing bicyclo ring,
R₄ is a hydrogen atom or a monovalent organic group, and
R₅⁻ is a counter anion.

In the above formula (b1) and above formula (b2), R₁, R₂ and R₃ are preferably each independently a C₁₋₂₀ alkyl group (e.g., methyl, ethyl, isopropyl, stearyl), a C₁₋₆ alkyl-carbonyl group (e.g., acetyl), a C₆₋₁₀ aryl group (e.g., phenyl), or an aromatic heterocyclic group (e.g., pyridyl), or R₁ and R₂ are taken together to form a nitrogen-containing heterocycle (e.g., piperazine) optionally substituted by C₁₋₆ alkyl group(s), or R₁, R₂ and R₃ are taken together to form a nitrogen-containing bicyclo ring (e.g., 1,8-diazabicyclo[5.4.0]-7-undecene, 1,4-diazabicyclo[2.2.2]octane).

In the above formula (b2), R₄ is preferably a hydrogen atom or a C₁₋₆ alkyl group.

In the above formula (b2), R₅⁻ is preferably a halide ion (e.g., a fluoride ion (F⁻)) or a hydroxide ion (OH⁻).

Preferable specific examples of the above amine compound include the following compounds.

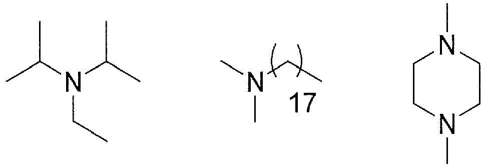

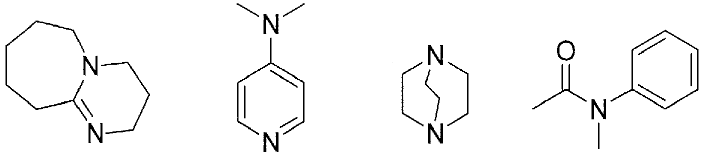

Preferable specific examples of the above ammonium compound include the following compounds.

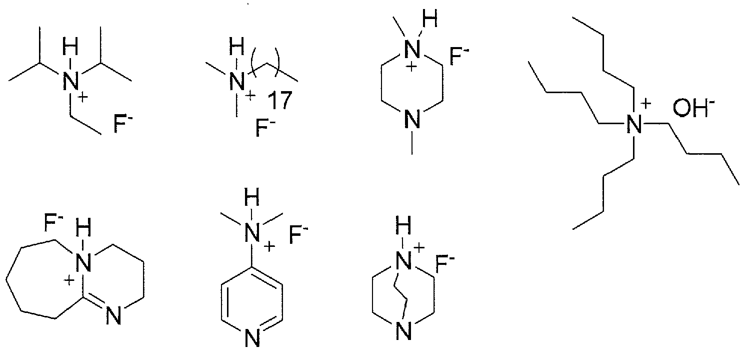

The imine compound is, for example, a compound represented by the following formula (b3). 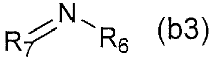 wherein
R₆ is a hydrogen atom or a monovalent organic group, and
R₇ is a divalent organic group, or
R₆ and R₇ are optionally taken together to form an optionally substituted unsaturated nitrogen-containing heterocycle.

The iminium compound is, for example, a compound represented by the following formula (b4). 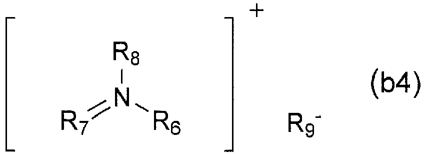 wherein
R₆ and R₈ are each independently a hydrogen atom or a monovalent organic group,
R₇ is a divalent organic group, or
R₆ and R₇ are optionally taken together to form an optionally substituted unsaturated nitrogen-containing heterocycle, and R₉⁻ is a counter anion.

In the above formula (b3) and above formula (b4), preferably, R₆ is a hydrogen atom, and R₇ is a C₁₋₆ alkylidene group (e.g., methylidene) optionally substituted by amino group(s), or R₆ and R₇ are taken together to form an unsaturated nitrogen-containing heterocycle (e.g., pyridine, pyrimidine, quinoline, octahydropyrimido[1,2-a]azepine) optionally substituted by C₁₋₆ alkyl group (s) or di-C₁₋₆ alkylamino group(s).

In the above formula (b4), R₈ is preferably a hydrogen atom.

In the above formula (b4), R₉⁻ is preferably a halide ion (e.g., a fluoride ion (F⁻)).

Preferable specific examples of the above imine compound include the following compounds.

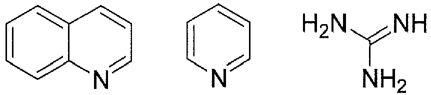

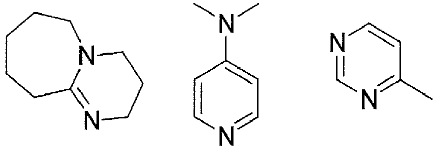

Preferable specific examples of the above iminium compound include the following compounds.

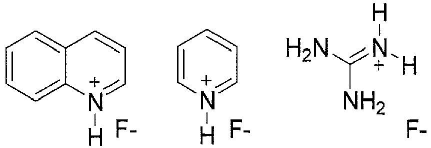

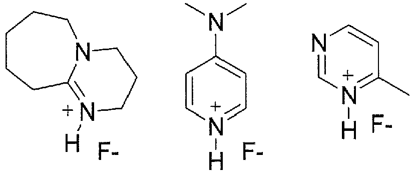

Examples of the N atom-free base include metal alkoxides and inorganic bases.

The metal alkoxide is, for example, represented by R₁₀O-M wherein R₁₀ is a C₁₋₆ alkyl group, and M is an alkali metal. Examples thereof include potassium tert-butoxide, sodium tert-butoxide, cesium tert-butoxide, potassium n-butoxide, sodium n-butoxide, cesium n-butoxide and the like.

Examples of the inorganic base include halide salts such as potassium fluoride, sodium fluoride, cesium fluoride and the like; hydroxide salts such as calcium hydroxide, aluminium hydroxide, potassium hydroxide, sodium hydroxide and the like; phosphorates such as disodium hydrogen phosphorate, sodium dihydrogen phosphorate and the like; carbonates such as potassium carbonate and the like; hydrogencarbonates such as sodium hydrogencarbonate and the like; acetates such as sodium acetate and the like; and the like.

The base is appropriately selected depending on the type of N-oxido aromatic heterocyclic compound (Ia).

The amount of the base to be used varies depending on the type of the base, and it is generally 1 to 10 mol, preferably 1 to 5 mol, per 1 mol of N-oxido aromatic heterocyclic compound (Ia) .

The reaction is carried out in a solvent selected from an aromatic hydrocarbon solvent, an ester solvent and an ether solvent. The use of such solvent allows good dissolution of tetrafluoroethylene and leads to the production of difluoromethyl-substituted aromatic heterocyclic compounds (IIa) with few by-products in good yield.

Examples of the aromatic hydrocarbon solvent include toluene, xylene, nitrobenzene and the like.

Examples of the ester solvent include butyl acetate, octyl acetate and the like.

Examples of the ether solvent include dibutyl ether, cyclopentyl methyl ether and the like.

The solvent used in the reaction is preferably a solvent having a boiling point of 80°C or higher, more preferably a solvent having a boiling point of 100°C or higher, particularly preferably a solvent having a boiling point of 110°C or higher, from the aspect of the reaction temperature. Examples of such solvents include toluene, xylene, nitrobenzene, butyl acetate, octyl acetate, dibutyl ether, cyclopentyl methyl ether and the like. Among them, the preferred are toluene, xylene and butyl acetate.

The amount of the solvent to be used is generally 100 to 1000 times, preferably 100 to 200 times the volume of N-oxido aromatic heterocyclic compound (Ia).

The reaction may be carried out in a flow system while flowing tetrafluoroethylene into a mixture of N-oxido aromatic heterocyclic compound (Ia), a proton source and a solvent. Alternatively, the reaction may be carried out in a sealed system after flowing tetrafluoroethylene into a mixture of N-oxido aromatic heterocyclic compound (Ia), a proton source and a solvent. The reaction is carried out preferably in a sealed system, more preferably under pressure in the range of 0.1 to 100.0 MPa, particularly preferably in the range of 0.1 to 10.0 MPa, from the aspect of the reaction efficiency, yield and reduction of by-products.

When N-oxido aromatic heterocyclic compound (Ia) is represented by the formula (IA), and R^{1a} and R^{1b}, R^{1b} and R^{1c}, or R^{1c} and R^{1d} are taken together to form the above C₆₋₁₀ arene, then the reaction is carried out preferably under pressure in the range of 0.1 to 10.0 MPa, more preferably in the range of 0.1 to 1.0 MPa, particularly preferably in the range of 0.1 to 0.5 MPa, from the aspect of ease of the reaction progress and increase in the reaction rate.

On the other hand, when N-oxido aromatic heterocyclic compound (Ia) is represented by the formula (IA), and does not form the above C₆₋₁₀ arene, then the reaction may be carried out under pressure in the above range. From the aspect of reduce in the reaction time, the reaction may be carried out preferably under pressure in the range of 0.1 to 30.0 MPa, more preferably in the range of 1.5 to 3.0 MPa, particularly preferably in the range of 2.0 to 2.5 MPa.

Prior to the inflow of tetrafluoroethylene, the reaction system is preferably degassed in advance. The reaction is also carried out preferably under nitrogen atmosphere.

The reaction is carried out preferably in the presence of a buffer solution with pH 5.0 to 8.0, more preferably in the presence of a buffer solution with pH 5.0 to 7.0. The use of such buffer solution reduces the formation of by-products and improves the yield. Examples of the buffer solution include phosphate buffer solutions (pH 7.0 to 7.5). For example, in the case of phosphate buffer solutions (pH 7.0-7.5), the amount of the buffer solution to be used is preferably 0.05 to 0.5 times the volume of the above solvent.

The reaction is carried out generally at 100°C or higher, preferably at temperature in the range of 100 to 300°C.

When N-oxido aromatic heterocyclic compound (Ia) is represented by the formula (IA), and R^{1a} and R^{1b}, R^{1b} and R^{1c}, or R^{1c} and R^{1d} are taken together to form the above C₆₋₁₀ arene, then the reaction is carried out preferably at temperature in the range of 100 to 200°C, particularly preferably in the range of 100 to 150°C, from the aspect of ease of the reaction progress.

On the other hand, when N-oxido aromatic heterocyclic compound (Ia) is represented by the formula (IA), and does not form the above C₆₋₁₀ arene, then the reaction may be carried out at temperature in the above range. From the aspect of ease of the reaction progress, the reaction may be carried out preferably at temperature in the range of 200 to 300°C, particularly preferably in the range of 200 to 250°C.

The reaction time varies depending on the type of N-oxido aromatic heterocyclic compound (Ia) and the reaction temperature, and it is generally 12 to 120 hr, preferably 24 to 48 hr.

After the completion of the reaction, the objective difluoromethyl-substituted aromatic heterocyclic compound (IIa) can be isolated and purified from the reaction mixture according to conventional method, for example, a separation means such as concentration, crystallization, recrystallization, distillation, solvent extraction, fractional distillation, column chromatography and the like.

The container used for the reaction of tetrafluoroethylene and N-oxido aromatic heterocyclic compound (Ia) is not limited as long as it does not adversely affect the reaction. For example, metal containers and the like can be used. Since olefin in a gaseous state under the reaction condition is handled in the present invention, the use of airsealing, pressure-resistant containers is preferable. Moreover, since the compound generated as the reaction progresses may become a reaction inhibitor by reacting with the metal of the reaction container, the use of containers lined with resin such as PFA resin (tetrafluoroethyleneperfluoroalkoxy ethylene copolymer) or lined with glass is preferable.

Difluoromethyl-substituted aromatic heterocyclic compound (IIa) thus obtained is useful as a synthetic intermediate for pharmaceutical or agrochemical products, and can be led to various pharmaceutical or agrochemical products.

Hereinafter, the production method of the present invention (Invention b) is explained.

In the present invention, aromatic heterocycle-substituted difluoroacetic acid derivative (IIIb) is produced by reacting N-oxido aromatic heterocyclic compound (Ib) with tetrafluoroethylene in the presence of compound (IIb) and a base, in a solvent selected from an aromatic hydrocarbon solvent, an ester solvent and an ether solvent. The reaction mechanism is as follows.

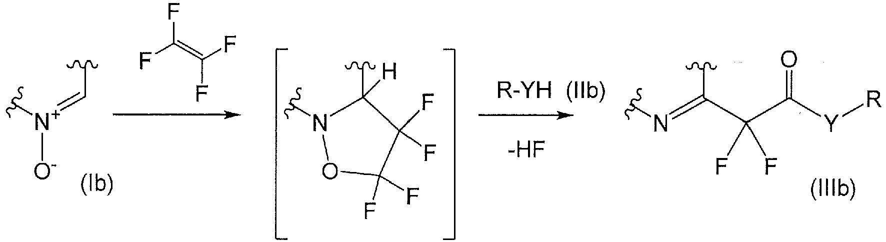

Specific examples of N-oxido aromatic heterocyclic compound (Ib) include the following N-oxido aromatic heterocyclic compounds (IA) and (IB): 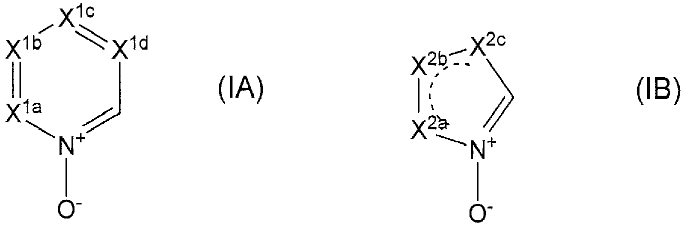 wherein
X^{1a} is CR^{1a} or N;
X^{1b} is CR^{1b} or N;
X^{1c} is CR^{1c} or N;
X^{1d} is CR^{1d} or N;
R^{1a}, R^{1b}, R^{1c} and R^{1d} are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group, or R^{1a} and R^{1b}, R^{1b} and R^{1c}, or R^{1c} and R^{1d} are optionally taken together to form a C₆₋₁₀ arene optionally substituted by substituent(s) selected from a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group;
X^{2a} is CR^{2a}, N, NR^{2a}, S or O;
X^{2b} is CR^{2b} or N;
X^{2c} is CR^{2c}, N, NR^{2c}, S or O; and
R^{2a}, R^{2b} and R^{2c} are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group, or R^{2a} and R^{2b}, or R^{2b} and R^{2c} are optionally taken together to form a C₆₋₁₀ arene optionally substituted by substituent(s) selected from a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group.

R^{1a}, R^{1b}, R^{1c} and R^{1d} are preferably each independently a hydrogen atom, a cyano group or a C₁₋₆ alkyl group, or R^{1a} and R^{1b}, R^{1b} and R^{1c}, or R^{1c} and R^{1d} are optionally taken together to form a C₆₋₁₀ arene optionally substituted by C₁₋₆ alkoxy-carbonyl group (s) .

R^{2a}, R^{2b} and R^{2c} are preferably each independently a hydrogen atom, a C₁₋₆ alkyl group or a C₇₋₁₆ aralkyl group, or R^{2a} and R^{2b}, or R^{2b} and R^{2c} are optionally taken together to form a C₆₋₁₀ arene.

In the formula (IB), one of the bond R^{2a}-R^{2b} and the bond R^{2b}-R^{2c} is a single bond, and the other is a double bond. Provided that when X^{2a} is S or O, then the bond R^{2a}-R^{2b} is a single bond, and the bond R^{2b}-R^{2c} is a double bond. Also, provided that when X^{2c} is S or O, then the bond R^{2a}-R^{2b} is a double bond, and the bond R^{2b}-R^{2c} is a single bond.

Specific examples of N-oxido aromatic heterocyclic compound (IA) include the following N-oxido aromatic heterocyclic compounds (IA-a) - (IA-e).

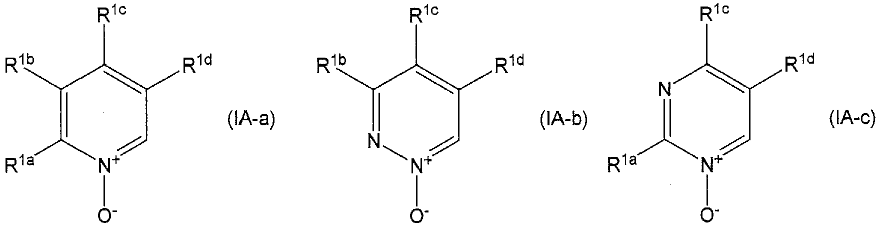

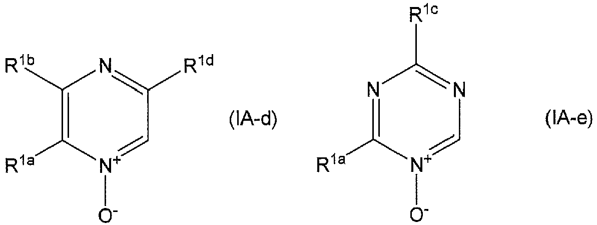

wherein each symbol in the formula is as defined above.

Among them, the preferred are (IA-a), (IA-b) and (IA-d).

Preferable specific examples of N-oxido aromatic heterocyclic compound (IA) include the following compounds. 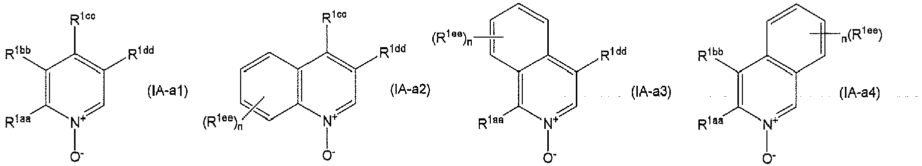 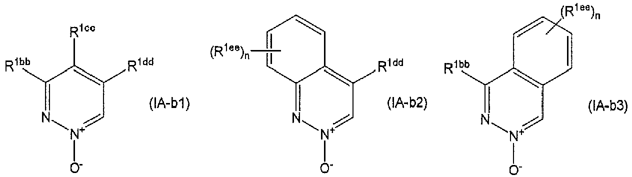 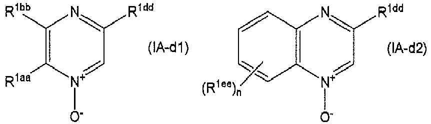 wherein
R^{1aa}, R^{1bb}, R^{1cc} and R^{1dd} are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group;
R^{1ee} in the number of n are each independently a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group; and
n is an integer of 0 to 4.
   R^{1aa}, R^{1bb}, R^{1cc} and R^{1dd} are preferably each independently a hydrogen atom, a cyano group or a C₁₋₆ alkyl group;
R^{1ee} in the number of n are preferably each independently a C₁₋₆ alkoxy-carbonyl group; and
n is preferably 0 or 1.

Specific examples of N-oxido aromatic heterocyclic compound (IB) include the following N-oxido aromatic heterocyclic compounds (IB-a) - (IB-ff). 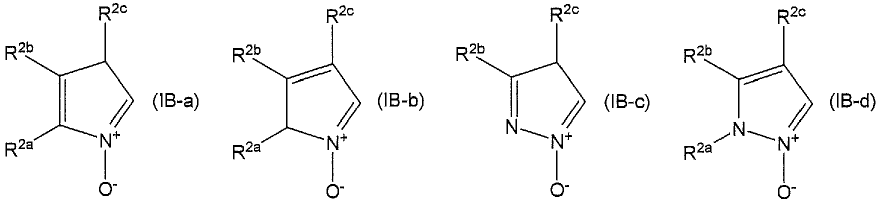 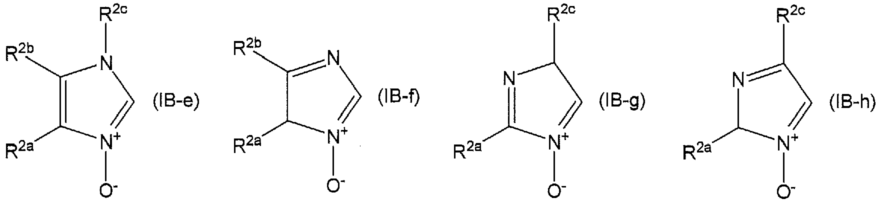 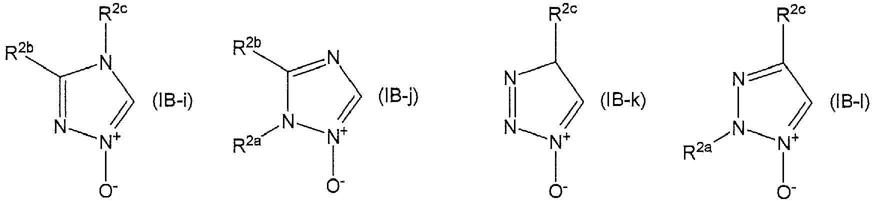 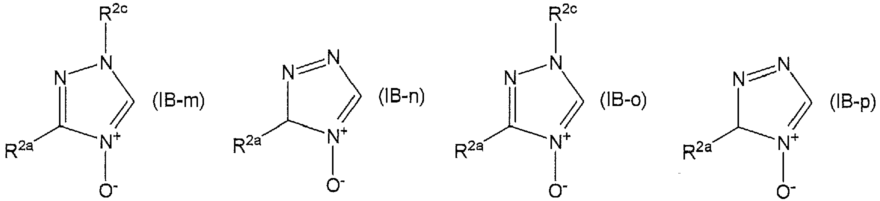 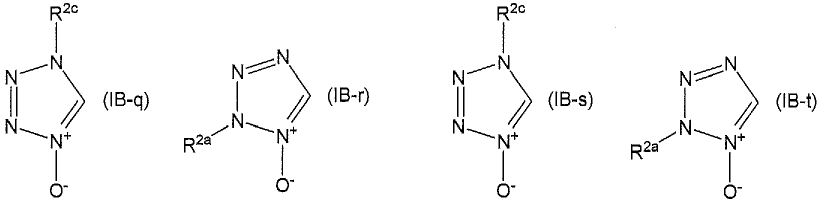 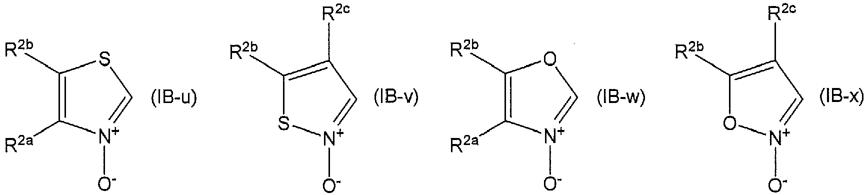 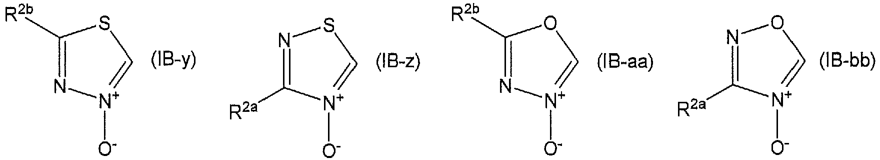 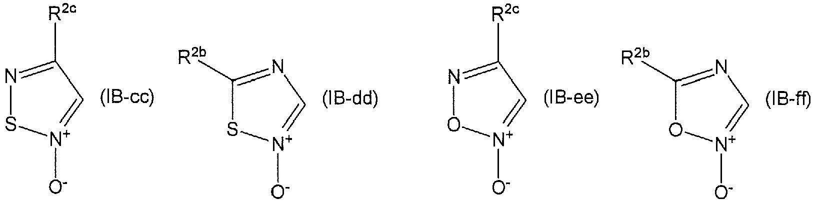 wherein each symbol in the formula is as defined above.

Among them, the preferred are (IB-e), (IB-u) and (IB-w).

Preferable specific examples of N-oxido aromatic heterocyclic compound (IB) include the following compounds. 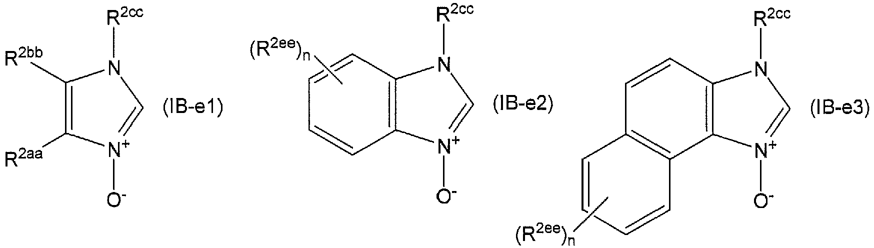 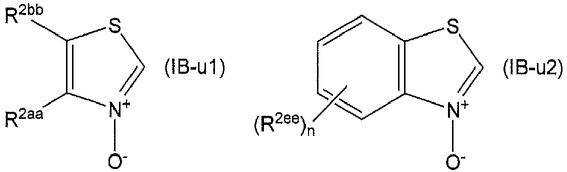 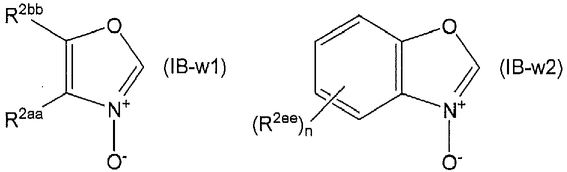 wherein
R^{2aa}, R^{2bb} and R^{2cc} are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group;
R^{2ee} in the number of n are each independently a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group; and
n is an integer of 0 to 4.

R^{2aa}, R^{2bb} and R^{2cc} are preferably each independently a hydrogen atom, a C₁₋₆ alkyl group or a C₇₋₁₆ aralkyl group; and n is preferably 0.

N-Oxido aromatic heterocyclic compound (Ib) may be commercially available, or can be produced according to a method known per se.

Tetrafluoroethylene (CF₂=CF₂) can be produced according to a known method. Since tetrafluoroethylene is in a gaseous state at room temperature under atmospheric pressure, it is added to the reaction system by inflow. In this case, tetrafluoroethylene diluted with nitrogen gas may be flowed into the reaction system. The amount of the tetrafluoroethylene to be used is generally 1 to 100 mol, preferably 1 to 10 mol, per 1 mol of N-oxido aromatic heterocyclic compound (Ib).

The reaction is carried out in the presence of compound (IIb). R in compound (IIb) is preferably a C₁₋₆ alkyl group optionally substituted by substituent(s) selected from a halogen atom, a cyano group, a C₁₋₆ alkoxy group and a C₁₋₆ haloalkoxy group; a C₇₋₁₆ aralkyl group or a C₆₋₁₀ aryl group, which of each is optionally substituted by substituent(s) selected from a halogen atom, a cyano group, a C₁₋₆ alkyl group, a C₁₋₆ haloalkyl group, a C₁₋₆ alkoxy group and a C₁₋₆ haloalkoxy group, more preferably a C₁₋₆ alkyl group.

Y in compound (IIb) is preferably O or NR^{Y}, more preferably O.

R^{Y} in compound (IIb) is preferably a hydrogen atom or a C₁₋₆ alkyl group, or R^{Y} and R are taken together to form a 5- or 6-membered nitrogen-containing heterocycle (e.g., morpholine) with the nitrogen atom to which they are bond.

Preferable specific examples of compound (IIb) include butanol, morpholine and the like.

The amount of compound (IIb) to be used can be appropriately selected, and is preferably 0.05 to 0.5 times the volume of the solvent described below.

The reaction is carried out in the presence of a base.

Through studies conducted by the present inventors, it has been found that a side reaction occurs during the following reaction in the above-mentioned reaction mechanism.

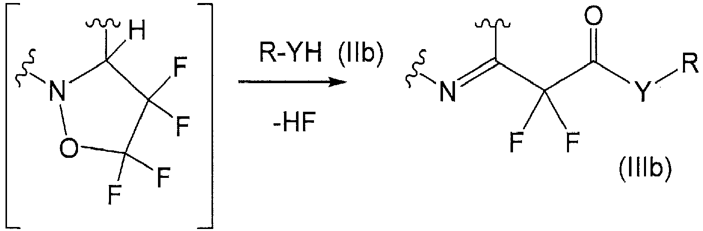

In order to solve this problem, in the present invention, a base is present in the reaction system to promote the withdrawal of protons from the above intermediate. This improves the reaction rate, reduces side reactions, and improves the yield.

As used herein, the base is a base capable of withdrawing a proton from the above intermediate, and examples of such base include a Brønsted base. It may be one that can be converted into a Bronsted base in the reaction system.

The above bases are broadly classified into N atom-containing bases and N atom-free bases.

Examples of the N atom-containing base include amine compounds, ammonium compounds, imine compounds, and iminium compounds.

The amine compound is, for example, a compound represented by the following formula (b1). 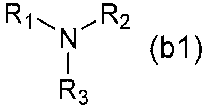 wherein
R₁, R₂ and R₃ are each independently a monovalent organic group, or R₁ and R₂ are optionally taken together to form an optionally substituted nitrogen-containing heterocycle, or R₁, R₂ and R₃ are optionally taken together to form a nitrogen-containing bicyclo ring.

The ammonium compound is, for example, a compound represented by the following formula (b2). 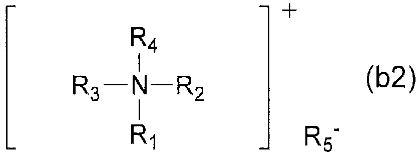 wherein
R₁, R₂ and R₃ are each independently a monovalent organic group, or R₁ and R₂ are optionally taken together to form an optionally substituted nitrogen-containing heterocycle, or R₁, R₂ and R₃ are optionally taken together to form a nitrogen-containing bicyclo ring,
R₄ is a hydrogen atom or a monovalent organic group, and
R₅⁻ is a counter anion.

In the above formula (b1) and above formula (b2), R₁, R₂ and R₃ are preferably each independently a C₁₋₂₀ alkyl group (e.g., methyl, ethyl, isopropyl, stearyl), a C₁₋₆ alkyl-carbonyl group (e.g., acetyl), a C₆₋₁₀ aryl group (e.g., phenyl), or an aromatic heterocyclic group (e.g., pyridyl), or R₁ and R₂ are taken together to form a nitrogen-containing heterocycle (e.g., piperazine) optionally substituted by C₁₋₆ alkyl group(s), or R₁, R₂ and R₃ are taken together to form a nitrogen-containing bicyclo ring (e.g., 1,8-diazabicyclo[5.4.0]-7-undecene, 1,4-diazabicyclo[2.2.2]octane).

In the above formula (b2), R₄ is preferably a hydrogen atom or a C₁₋₆ alkyl group.

In the above formula (b2), R₅⁻ is preferably a halide ion (e.g., a fluoride ion (F⁻)) or a hydroxide ion (OH⁻).

Preferable specific examples of the above amine compound include the following compounds.

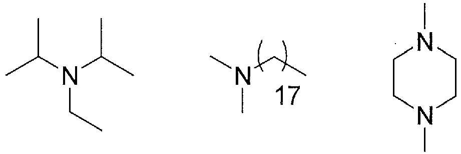

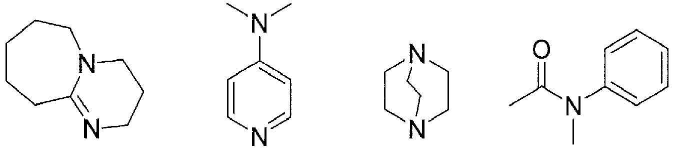

Preferable specific examples of the above ammonium compound include the following compounds.

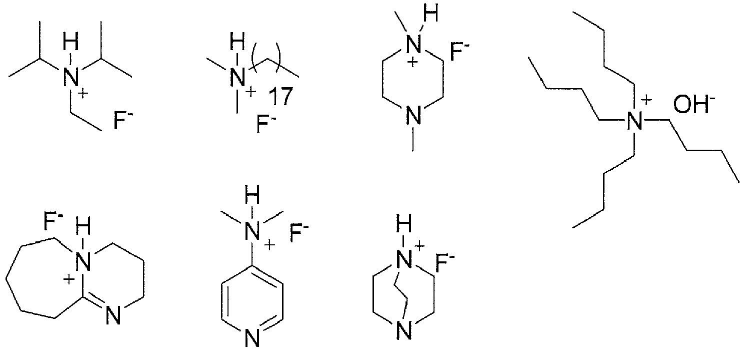

The imine compound is, for example, a compound represented by the following formula (b3). 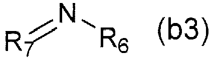 wherein
R₆ is a hydrogen atom or a monovalent organic group, and
R₇ is a divalent organic group, or
R₆ and R₇ are optionally taken together to form an optionally substituted unsaturated nitrogen-containing heterocycle.

The iminium compound is, for example, a compound represented by the following formula (b4). 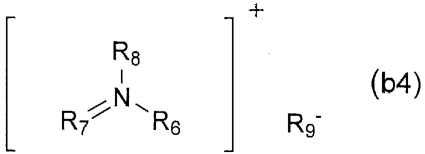 wherein
R₆ and R₈ are each independently a hydrogen atom or a monovalent organic group,
R₇ is a divalent organic group, or
R₆ and R₇ are optionally taken together to form an optionally substituted unsaturated nitrogen-containing heterocycle, and R₉⁻ is a counter anion.

In the above formula (b3) and above formula (b4), preferably, R₆ is a hydrogen atom, and R₇ is a C₁₋₆ alkylidene group (e.g., methylidene) optionally substituted by amino group(s), or R₆ and R₇ are taken together to form an unsaturated nitrogen-containing heterocycle (e.g., pyridine, pyrimidine, quinoline, octahydropyrimido[1,2-a]azepine) optionally substituted by C₁₋₆ alkyl group (s) or di-C₁₋₆ alkylamino group(s).

In the above formula (b4), R₈ is preferably a hydrogen atom.

In the above formula (b4), R₉⁻ is preferably a halide ion (e.g., a fluoride ion (F⁻)).

Preferable specific examples of the above imine compound include the following compounds.

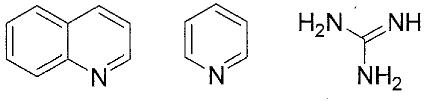

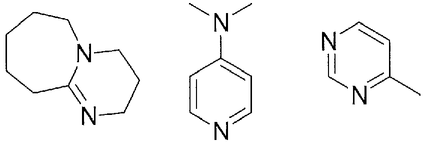

Preferable specific examples of the above iminium compound include the following compounds.

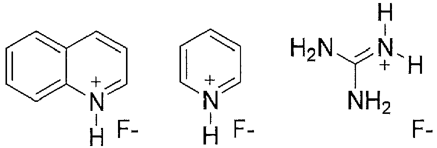

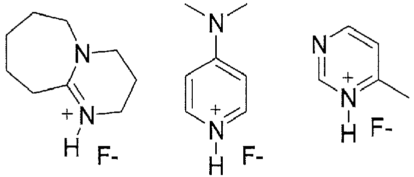

Examples of the N atom-free base include metal alkoxides and inorganic bases.

The metal alkoxide is, for example, represented by R₁₀O-M wherein R₁₀ is a C₁₋₆ alkyl group, and M is an alkali metal. Examples thereof include potassium tert-butoxide, sodium tertbutoxide, cesium tert-butoxide, potassium n-butoxide, sodium n-butoxide, cesium n-butoxide and the like.

Examples of the inorganic base include halide salts such as potassium fluoride, sodium fluoride, cesium fluoride and the like; hydroxide salts such as calcium hydroxide, aluminium hydroxide, potassium hydroxide, sodium hydroxide and the like; phosphorates such as disodium hydrogen phosphorate, sodium dihydrogen phosphorate and the like; carbonates such as potassium carbonate and the like; hydrogencarbonates such as sodium hydrogencarbonate and the like; acetates such as sodium acetate and the like; and the like.

The base is appropriately selected depending on the type of N-oxido aromatic heterocyclic compound (Ib).

The amount of the base to be used varies depending on the type of the base, and it is generally 1 to 10 mol, preferably 1 to 5 mol, per 1 mol of N-oxido aromatic heterocyclic compound (Ib) .

The reaction is carried out in a solvent selected from an aromatic hydrocarbon solvent, an ester solvent and an ether solvent. The use of such solvent allows good dissolution of tetrafluoroethylene and leads to the production of aromatic heterocycle-substituted difluoroacetic acid derivative (IIIb) with few by-products.

Examples of the aromatic hydrocarbon solvent include toluene, xylene, nitrobenzene and the like.

Examples of the ester solvent include butyl acetate, octyl acetate and the like.

Examples of the ether solvent include dibutyl ether, cyclopentyl methyl ether and the like.

The solvent used in the reaction is preferably a solvent having a boiling point of 80°C or higher, more preferably a solvent having a boiling point of 100°C or higher, particularly preferably a solvent having a boiling point of 110°C or higher, from the aspect of the reaction temperature. Examples of such solvents include toluene, xylene, nitrobenzene, butyl acetate, octyl acetate, dibutyl ether, cyclopentyl methyl ether and the like. Among them, the preferred are toluene and butyl acetate.

The amount of the solvent to be used is generally 100 to 1000 times, preferably 100 to 200 times the volume of N-oxido aromatic heterocyclic compound (Ib).

The reaction may be carried out in a flow system while flowing tetrafluoroethylene into a mixture of N-oxido aromatic heterocyclic compound (Ib), compound (IIb) and a solvent. Alternatively, the reaction may be carried out in a sealed system after flowing tetrafluoroethylene into a mixture of N-oxido aromatic heterocyclic compound (Ib), compound (IIb) and a solvent. The reaction is carried out preferably in a sealed system, more preferably under pressure in the range of 0.1 to 100.0 MPa, particularly preferably in the range of 0.1 to 10.0 MPa, from the aspect of the reaction efficiency and reduction of by-products.

When N-oxido aromatic heterocyclic compound (Ib) is represented by the formula (IA), and R^{1a} and R^{1b}, R^{1b} and R^{1c}, or R^{1c} and R^{1d} are taken together to form the above C₆₋₁₀ arene, then the reaction is carried out preferably under pressure in the range of 0.1 to 10.0 MPa, more preferably in the range of 0.1 to 1.0 MPa, particularly preferably in the range of 0.1 to 0.5 MPa, from the aspect of ease of the reaction progress and increase in the reaction rate.

On the other hand, when N-oxido aromatic heterocyclic compound (Ib) is represented by the formula (IA), and does not form the above C₆₋₁₀ arene, then the reaction may be carried out under pressure in the above range. From the aspect of ease of the reaction progress and increase in the reaction rate, the reaction may be carried out preferably under pressure in the range of 0.1 to 30.0 MPa, more preferably in the range of 1.5 to 3.0 MPa, particularly preferably in the range of 2.0 to 2.5 MPa.

Prior to the inflow of tetrafluoroethylene, the reaction system is preferably degassed in advance. The reaction is also carried out preferably under nitrogen atmosphere.

The reaction is carried out generally at 100°C or higher, preferably at temperature in the range of 100 to 300°C.

When N-oxido aromatic heterocyclic compound (Ib) is represented by the formula (IA), and R^{1a} and R^{1b}, R^{1b} and R^{1c}, or R^{1c} and R^{1d} are taken together to form the above C₆₋₁₀ arene, then the reaction is carried out preferably at temperature in the range of 100 to 200°C, particularly preferably in the range of 100 to 150°C, from the aspect of ease of the reaction progress and increase in the reaction rate.

On the other hand, when N-oxido aromatic heterocyclic compound (Ib) is represented by the formula (IA), and does not form the above C₆₋₁₀ arene, then the reaction may be carried out at temperature in the above range. From the aspect of ease of the reaction progress and increase in the reaction rate, the reaction may be carried out preferably at temperature in the range of 200 to 300°C, particularly preferably in the range of 200 to 250°C.

The reaction time varies depending on the types of N-oxido aromatic heterocyclic compound (Ib) and compound (IIb), and the reaction temperature, and it is generally 12 to 120 hr, preferably 24 to 48 hr.

After the completion of the reaction, the objective aromatic heterocycle-substituted difluoroacetic acid derivative (IIIb) can be isolated and purified from the reaction mixture according to conventional method, for example, a separation means such as concentration, crystallization, recrystallization, distillation, solvent extraction, fractional distillation, column chromatography and the like.

The container used for the reaction of tetrafluoroethylene and N-oxido aromatic heterocyclic compound (Ib) is not limited as long as it does not adversely affect the reaction. For example, metal containers and the like can be used. Since olefin in a gaseous state under the reaction condition is handled in the present invention, the use of air-sealing, pressure-resistant containers is preferable. Moreover, since the compound generated as the reaction progresses may become a reaction inhibitor by reacting with the metal of the reaction container, the use of containers lined with resin such as PFA resin (tetrafluoroethylene-perfluoroalkoxy ethylene copolymer) or lined with glass is preferable.

For aromatic heterocycle-substituted difluoroacetic acid derivative (IIIb) thus obtained, for example, aromatic heterocycle-substituted difluoroacetic acid derivative (IIIb) wherein Y is O can be led to a difluoromethyl-substituted aromatic heterocyclic compound having a partial structure represented by the formula (IVb): 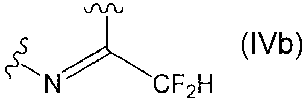 which is useful as a synthetic intermediate for pharmaceutical or agrochemical products, by a known method, for example, hydrolysis, followed by decarboxylation.

### Examples

The present invention is explained in detail in the following by referring to Examples, which are not to be construed as limitative.

### [Analysis Method]

Nuclear magnetic resonance spectrum (NMR) was measured using JNM-AL300 manufactured by JEOL Ltd. ¹H NMR was measured at 300MHz using tetramethylsilane as standard.

Mass spectrometry (LC-MS) was measured using liquid chromatograph mass spectrum system (LCMS6120B) manufactured by Agilent Technologies. Mass spectrometry (GC-MS) was determined by electron ionization (EI) using gas chromatograph mass spectrometer (GCMS-QP5000V2 or GCMS-QP2010Ultra) manufactured by Shimadzu Corporation. Quantitative analysis (GC) of yield was performed by a gas chromatograph system (Agilent 6850) manufactured by Agilent Technologies, using a column of DB-1, 60 m, diameter 0.25 mm, and film thickness 1 um.

### [Example 1]

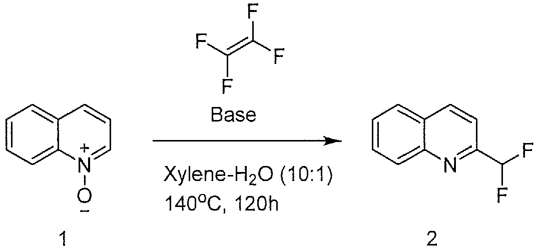

A catalyst screening container (model HIP-7506) manufactured by SynFlex was used as a reaction container. Xylene (10 ml) and water (1 ml) were added thereto, and the mixture was stirred and kept at 25°C. Then, quinoline N-oxide (109 mg, 0.751 mmol, Compound 1) and various bases (0.751 mmol) were added thereto, and the mixture was degassed under reduced pressure. Tetrafluoroethylene diluted to 50% with nitrogen gas was flowed into the reactor until the reactor pressure reached 0.2 MPa, and then the mixture was continuously stirred under heating at 140°C for 120 hr. By measuring NMR, GC and GC-MS of the crude solution after the completion of the reaction, the yield of Compound 2 was calculated.

¹H-NMR and GC-MS of Compound 2 are shown below.
¹H-NMR(CDCl₃) δ 8.34 (d, 1H), 8.15 (d, 1H), 7.90 (d, 1H), 7.80 (dd, 1H), 7.74 (d, 1H), 7.65 (dd, 1H), 6.79 (t, 1H)
GC-MS(EI):[M+]=179

Table 1 below shows the yield (%) of Compound 2 for each base, along with the experimental result of base-free control for comparison.

**[Table 1]**

| | | | | | | | | |
|---|---|---|---|---|---|---|---|---|
| | Control | | | | | | | |
| Yield(%) | 21 | | | | | | | |
| Base | NaF | KF | CsF | Al(OH)₃ | Ca(OH)₂ | NaOAc | NaHCO₃ | KOH |
| Yield(%) | 40 | 53 | 44 | 38 | 55 | 46 | 52 | 54 |
| Base | 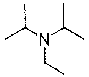 | 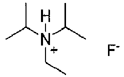 | 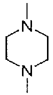 | 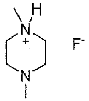 | 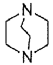 | 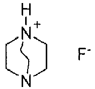 | 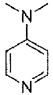 | 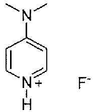 |
| Yield (%) | 80 | 79 | 60 | 59 | 45 | 42 | 43 | 41 |
| Base | 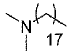 | 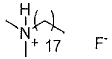 | 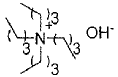 | | | | | |
| Yield (%) | 95 | 90 | 39 | | | | | |

### [Example 2]

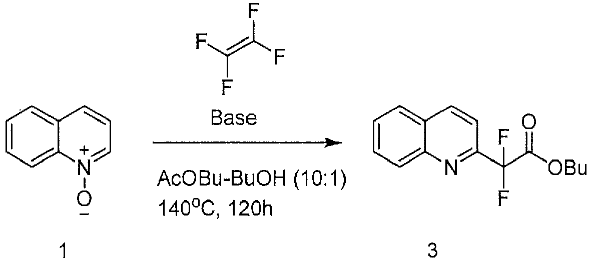

A crude solution containing Compound 3 was obtained from Compound 1 in the same manner as described in Example 1, except that xylene and water were replaced with butyl acetate and butanol. By measuring NMR, GC and GC-MS of the crude solution after the completion of the reaction, the yield of Compound 3 was calculated.

¹H-NMR and LC-MS of Compound 3 are shown below. ¹H-NMR(CDCl₃)δ 8.35 (d, 1H), 8.15 (d, 1H), 7.89 (d, 1H), 7.72-7.81(m, 2H), 7.63 (t, 1H), 4.36 (t, 2H), 1.67 (tt, 2H), 1.35 (tt, 2H), 0.88 (t, 3H)
LC-MS:[M+1]=280

Table 2 below shows the yield (%) of Compound 3 for each base, along with the experimental result of base-free control for comparison.

**[Table 2]**

| | | | | | | | | |
|---|---|---|---|---|---|---|---|---|
| | Control | | | | | | | |
| Yield(%) | 30 | | | | | | | |
| Base | NaF | KF | Al(OH)₃ | Ca(OH)₂ | NaHCO₃ | | | |
| Yield(%) | 43 | 64 | 63 | 74 | 60 | | | |
| Base | 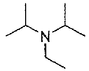 | 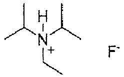 | 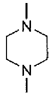 | 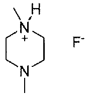 | 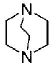 | 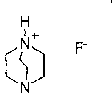 | 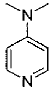 | 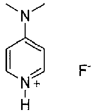 |
| Yield(%) | 48 | 49 | 41 | 42 | 45 | 42 | 43 | 41 |
| Base | 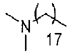 | 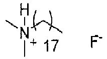 | | | | | | |
| Yield(%) | 52 | 53 | | | | | | |

### Industrial Applicability

According to the present invention (Invention a), by an easy and inexpensive method of flowing tetrafluoroethylene into N-oxido aromatic heterocyclic compound (Ia) in the presence of a base, in a specific solvent, difluoromethyl-substituted aromatic heterocyclic compound (IIa) with few by-products can be produced in good yield in one step.

According to the present invention (Invention b), by an easy and inexpensive method of flowing tetrafluoroethylene into N-oxido aromatic heterocyclic compound (Ib) in the presence of compound (IIb) and a base, in a specific solvent, aromatic heterocycle-substituted difluoroacetic acid derivative (IIIb) with few by-products can be produced in good yield in one step.

This application is based on patent application No. 2021-131010 filed on August 11, 2021 in Japan, the contents of which are encompassed in full herein.

## Claims

1. A method for producing a difluoromethyl-substituted aromatic heterocyclic compound having a partial structure represented by the formula (IIa): 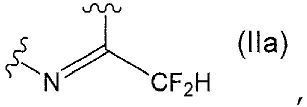 which comprises reacting an N-oxido aromatic heterocyclic compound having a partial structure represented by the formula (Ia) : 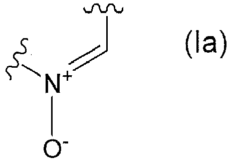 with tetrafluoroethylene in the presence of a base, in a solvent selected from an aromatic hydrocarbon solvent, an ester solvent and an ether solvent.

2. The method according to Claim 1, wherein the reaction is carried out at temperature in the range of 100 to 300°C.

3. The method according to Claim 1 or 2, wherein the reaction is carried out under pressure in the range of 0.1 to 10.0 MPa.

4. The method according to Claim 1, wherein the reaction is carried out in the presence of a buffer solution with pH 5.0 to 8.0.

5. The method according to Claim 1, wherein the N-oxido aromatic heterocyclic compound having the partial structure represented by the formula (Ia) is an N-oxido aromatic heterocyclic compound represented by the formula (IA) or (IB): 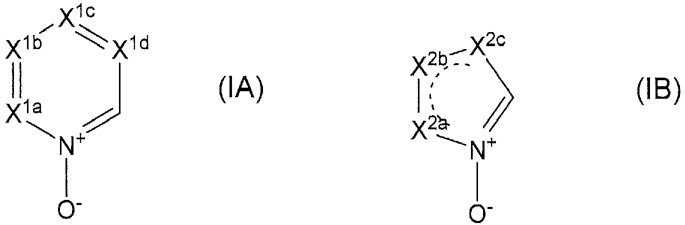 wherein
X^{1a} is CR^{1a} or N;
X^{1b} is CR^{1b} or N;
X^{1c} is CR^{1c} or N;
X^{1d} is CR^{1d} or N;
R^{1a}, R^{1b}, R^{1c} and R^{1d} are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group, or R^{1a} and R^{1b}, R^{1b} and R^{1c}, or R^{1c} and R^{1d} are optionally taken together to form a C₆₋₁₀ arene optionally substituted by substituent(s) selected from a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group;
X^{2a} is CR^{2a}, N, NR^{2a}, S or O;
X^{2b} is CR^{2b} or N;
X^{2c} is CR^{2c}, N, NR^{2c}, S or O; and
R^{2a}, R^{2b} and R^{2c} are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group, or R^{2a} and R^{2b}, or R^{2b} and R^{2c} are optionally taken together to form a C₆₋₁₀ arene optionally substituted by substituent(s) selected from a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group.

6. A method for producing an aromatic heterocycle-substituted difluoroacetic acid derivative having a partial structure represented by the formula (IIIb): 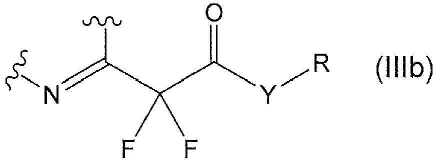 wherein
R is a C₁₋₈ alkyl group, a C₃₋₈ cycloalkyl group, a C₇₋₁₆ aralkyl group or a C₆₋₁₀ aryl group, wherein the C₁₋₈ alkyl group is optionally substituted by substituent(s) selected from a halogen atom, a cyano group, a C₁₋₆ alkoxy group and a C₁₋₆ haloalkoxy group, and the C₃₋₈ cycloalkyl group, C₇₋₁₆ aralkyl group and C₆₋₁₀ aryl group are each optionally substituted by substituent(s) selected from a halogen atom, a cyano group, a C₁₋₆ alkyl group, a C₁₋₆ haloalkyl group, a C₁₋₆ alkoxy group and a C₁₋₆ haloalkoxy group,
Y is O, S or NR^{Y}, and
R^{Y} is a hydrogen atom or a C₁₋₈ alkyl group, or R^{Y} and R are optionally taken together to form a nitrogen-containing heterocycle with the nitrogen atom to which they are bond, which comprises reacting an N-oxido aromatic heterocyclic compound having a partial structure represented by the formula (Ib) : 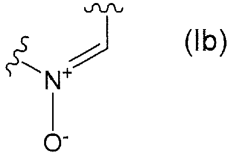 with tetrafluoroethylene in the presence of a compound represented by the formula (IIb): R-YH wherein each symbol in the formula is as defined above and a base, in a solvent selected from an aromatic hydrocarbon solvent, an ester solvent and an ether solvent.

7. The method according to Claim 6, wherein the reaction is carried out at temperature in the range of 100 to 300°C.

8. The method according to Claim 6 or 7, wherein the reaction is carried out under pressure in the range of 0.1 to 10.0 MPa.

9. The method according to Claim 6, wherein the N-oxido aromatic heterocyclic compound having the partial structure represented by the formula (Ib) is an N-oxido aromatic heterocyclic compound represented by the formula (IA) or (IB): 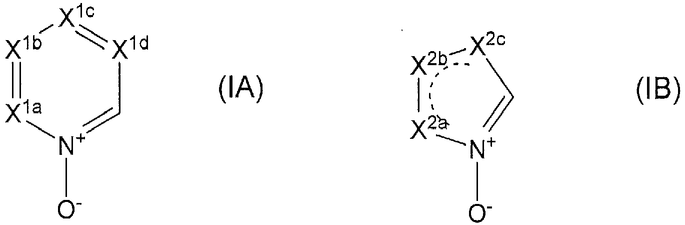 wherein
X^{1a} is CR^{1a} or N;
X^{1b} is CR^{1b} or N;
X^{1c} is CR^{1c} or N;
X^{1d} is CR^{1d} or N;
R^{1a}, R^{1b}, R^{1c} and R^{1d} are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group, or R^{1a} and R^{1b}, R^{1b} and R^{1c}, or R^{1c} and R^{1d} are optionally taken together to form a C₆₋₁₀ arene optionally substituted by substituent(s) selected from a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group;
X^{2a} is CR^{2a}, N, NR^{2a}, S or O;
X^{2b} is CR^{2b} or N;
X^{2c} is CR^{2c}, N, NR^{2c}, S or O; and
R^{2a}, R^{2b} and R^{2c} are each independently a hydrogen atom, a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group or a C₇₋₁₆ aralkyl group, or R^{2a} and R^{2b}, or R^{2b} and R^{2c} are optionally taken together to form a C₆₋₁₀ arene optionally substituted by substituent(s) selected from a halogen atom, a cyano group, a C₁₋₆ alkyl group, a C₁₋₆ haloalkyl group, a hydroxy group, a C₁₋₆ alkoxy group, a C₁₋₆ haloalkoxy group, a sulfanyl group, a C₁₋₆ alkylsulfanyl group, an amino group, a mono-C₁₋₆ alkylamino group, a di-C₁₋₆ alkylamino group, a formyl group, a carboxy group, a C₁₋₆ alkoxy-carbonyl group, a C₆₋₁₀ aryl group and a C₇₋₁₆ aralkyl group.
